# EUROPEAN PATENT APPLICATION

(11) **EP 4 393 507 A1**
(43) Date of publication of application: **03.07.2024**
(21) Application number: 22848863.1
(22) Date of filing: 10.02.2022
(51) Int. Cl.: A61K 45/00, A61K 31/155, A61K 31/395, A61K 31/42, A61K 31/5377, A61K 39/395, A61P 25/02, A61P 43/00

(54) **PHARMACEUTICAL COMPOSITION FOR TREATING PERIPHERAL NERVE INJURY**

(30) Priority: 30.07.2021 JP 2021126207
(71) Applicant: National University Corporation Hokkaido University, Sapporo-shi, Hokkaido 060-0808 (JP)
(72) Inventor: KADOYA Ken, Sapporo-shi, Hokkaido 060-0808 (JP); YAMAMOTO Yasuhiro, Sapporo-shi, Hokkaido 060-0808 (JP)
(74) Representative: Wasner, Marita
(86) International application number: PCT/JP2022/005504
(87) International publication number: WO 2023/007779

(57) **Abstract**

The present invention provides a pharmaceutical composition for treating peripheral nerve injury, said pharmaceutical composition comprising a substance that inhibits the formation of neutrophil extracellular traps (NETs) outside the parenchyma of injured peripheral nerves, for example, a substance that inhibits the accumulation of neutrophils in injured peripheral nerves, a substance that suppresses the expression or inhibits the function of macrophage migration inhibitory factor (MIF), etc. The present invention further provides a method for evaluating the peripheral nerve regeneration-promoting activity of a test substance using, as an indicator, the activity to inhibit the formation of NETs outside the parenchyma of injured peripheral nerves, for example, the activity to inhibit the accumulation of neutrophils in injured peripheral nerves, the activity to suppress the expression of MIF or the activity to inhibit the function thereof, etc.

## Description

### Field

The present invention relates to a pharmaceutical composition for treating peripheral nerve injury, containing a substance that suppresses the formation of neutrophil extracellular traps (NETs) outside the parenchyma of an injured peripheral nerve. The present invention further relates to a method for evaluating the peripheral nerve regeneration-promoting activity of a test substance by using its activity of suppressing NETs formation outside the parenchyma of an injured peripheral nerve as an indicator.

### Background

Treatments currently performed for peripheral nerve injuries due to trauma or other causes include follow-up observation, suturing, and reconstruction using autologous nerves or nerve regeneration-inducing tubes (artificial nerves). Autologous nerve grafting is the gold standard for peripheral nerve reconstruction. However, it involves a problem in that autologous nerves for grafting must be collected from healthy sites other than the injury sites. Therefore, the development of new methods for peripheral nerve regeneration that do not sacrifice autologous nerves is desired.

Although regeneration of peripheral nerve is more vigorous than that of the central nervous system, clinical outcomes in proximal injuries, severe injuries, and reconstructive cases are not favorable. Muscles tend to lose their sensitivity to nerve reinnervation 3 to 6 months after the loss of innervation, so it is important to regenerate axons as early as possible to reach the muscle and establish reinnervation in the treatment of peripheral nerve injuries. However, considering that the rate of axonal regeneration is usually 1-2 mm/day, proximal injuries that require axonal regeneration over long distances, generally 30 cm to 90 cm, lose their sensitivity to reinnervation by the time the regenerated axon reaches the muscle. In addition, more axons must be regenerated in severe injuries and in reconstructive cases.

The process from peripheral nerve injury to regeneration is as follows. When a peripheral nerve is injured and the axon is disrupted, Wallerian degeneration occurs distal to the injury, resulting in fragmentation of the axon and myelin. The fragmented axons and myelin are removed by the phagocytosis of Schwann cells and macrophages. Activated Schwann cells then align distal to the injury and provide a scaffold for axon regeneration, thereby promoting axon sprouting and elongation. Subsequently, nerve regeneration is completed by remyelination of the axon and reformation of synapses with the target organ.

In the treatment of peripheral nerve injury, particularly in the treatment of proximal injuries, severe injuries, and reconstructive cases with poor clinical outcomes, it is necessary to increase the rate and amount of axonal regeneration through control of the above nerve regeneration process. Although several methods to promote regeneration of injured peripheral nerves have been reported (for example, Patent Literature 1 and 2), none has yet reached clinical application.

### Citation List

### Patent Literature

Patent Literature 1: WO2018/056412
Patent Literature 2: WO2005/074655

### Summary

The present invention provides a new means for promoting axonal regeneration that can be used in the regenerative treatment of injured peripheral nerves.

The inventors of the present invention have found that neutrophils delay the regenerative process of injured peripheral nerves via NETs.

The present disclosure provides the followings:
Item 1. A pharmaceutical composition for treating peripheral nerve injury, containing a substance that suppresses the formation of neutrophil extracellular traps (NETs) outside the parenchyma of an injured peripheral nerve.
Item 2. The pharmaceutical composition according to item 1, wherein the substance that suppresses the formation of NETs outside the parenchyma of an injured peripheral nerve is a substance that inhibits the accumulation of neutrophils in an injured peripheral nerve, a substance that inhibits NETs formation by neutrophils, or a substance that degrades NETs.
Item 3. The pharmaceutical composition according to item 2, wherein the substance that inhibits the accumulation of neutrophils in an injured peripheral nerve is selected from the group consisting of an antibody against neutrophils and antigen-binding fragment thereof, an aptamer targeting neutrophils, an inhibitor of neutrophil migration stimulating factor, and a neutrophil migration inhibitory factor.
Item 4. The pharmaceutical composition according to item 2, wherein the substance that inhibits NETs formation by neutrophils is selected from the group consisting of a substance that suppresses the expression or inhibits the function of macrophage migration inhibitory factor (MIF), a substance that suppresses the expression or inhibits the function of CXC motif-type chemokine receptor 4 (CXCR4), and a substance that suppresses the expression or inhibits the function of peptidylarginine deiminase (PAD4).
Item 5. The pharmaceutical composition according to item 4, wherein the substance that suppresses the expression or inhibits the function of MIF is selected from the group consisting of a nucleic acid that suppresses the expression of MIF, an antibody against MIF and antigen-binding fragment thereof, an aptamer targeting MIF, and a MIF inhibitor.
Item 6. The pharmaceutical composition according to item 5, wherein the MIF inhibitor is ISO-1((S,R)-3-(4-Hydroxyphenyl)-4,5-dihydro-5-isoxazole acetic acid) or (±)-CPSI 1306(2-[3-(3-(2,4-Difluorophenyl)-4,5-dihydro-5-isoxazolyl]-1-(4-morpholinyl)ethanone).
Item 7. The pharmaceutical composition according to item 4, wherein the substance that suppresses the expression or inhibits the function of CXCR4 is selected from the group consisting of a nucleic acid that suppresses the expression of CXCR4, an antibody against CXCR4 and antigen-binding fragment thereof, an aptamer targeting CXCR4, and a CXCR4 inhibitor.
Item 8. The pharmaceutical composition according to item 7, wherein the CXCR4 inhibitor is plerixafol.
Item 9. The pharmaceutical composition according to item 4, wherein the substance that suppresses the expression or inhibits the function of PAD4 is selected from the group consisting of a nucleic acid that suppresses the expression of PAD4, an antibody against PAD4 and antigen-binding fragment thereof, an aptamer targeting PAD4, and a PAD4 inhibitor.
Item 10. The pharmaceutical composition according to item 9, wherein the PAD4 inhibitor is Cl-amidine.
Item 11. The pharmaceutical composition according to item 2, wherein the substance that degrades NETs is DNase I.
Item 12. The pharmaceutical composition according to any one of items 1-11, for use by placement in the distal vicinity of an injury site of a peripheral nerve.
Item 13. A method for evaluating the peripheral nerve regeneration-promoting activity of a test substance by using the activity of the substance of suppressing NETs formation outside the parenchyma of an injured peripheral nerve as an indicator.
Item 14. The method according to item 13, wherein the activity of the substance of suppressing NETs formation outside the parenchyma of an injured peripheral nerve is the activity of inhibiting the accumulation of neutrophils in an injured peripheral nerve, the activity of inhibiting NETs formation by neutrophils, or the activity of degrading NETs.
Item 15. The method according to item 14, wherein the activity of inhibiting NETs formation by neutrophils is selected from the group consisting of the activity of suppressing the expression or inhibiting the function of MIF, the activity of suppressing the expression or inhibiting the function of CXCR4, and the activity of suppressing the expression or inhibiting the function of PAD4.

The present disclosure further provides the followings:
Item 1A. A pharmaceutical composition for treating peripheral nerve injury, containing a substance that inhibits the accumulation of neutrophils in an injured peripheral nerve, or a substance that suppresses the expression or inhibits the function of MIF.
Item 2A. The pharmaceutical composition according to item 1, wherein the substance that inhibits the accumulation of neutrophils in an injured peripheral nerve is at least one substance selected from the group consisting of an antibody against neutrophils and antigen-binding fragment thereof, an inhibitor of neutrophil migration stimulating factor, and a neutrophil migration inhibitory factor.
Item 3A. The pharmaceutical composition according to item 1, wherein the substance that suppresses the expression or inhibits the function of MIF is at least one substance selected from the group consisting of a nucleic acid that suppresses the expression of MIF, an antibody against MIF and antigen-binding fragment thereof, and a MIF inhibitor.
Item 4A. The pharmaceutical composition according to item 3, wherein the MIF inhibitor is ISO-1((S,R)-3-(4-Hydroxyphenyl)-4,5-dihydro-5-isoxazole acetic acid) or (±)-CPSI 1306(2-[3-(3-(2,4-Difluorophenyl)-4,5-dihydro-5-isoxazolyl]-1-(4-morpholinyl)ethanone).
Item 5A. The pharmaceutical composition according to any one of items 1-4, for use by placement in the distal vicinity of an injury site of a peripheral nerve.
Item 6A. A method for evaluating the peripheral nerve regeneration-promoting activity of a test substance by using the activity of inhibiting the accumulation of neutrophils in an injured peripheral nerve as an indicator.
Item 7A. A method for evaluating the peripheral nerve regeneration-promoting activity of a test substance by using the activity of suppressing the expression or inhibiting the function of MIF as an indicator.

According to the present disclosure, effective treatment of peripheral nerve injury can be achieved through the promotion of axonal regeneration.

### Brief Description of Drawings

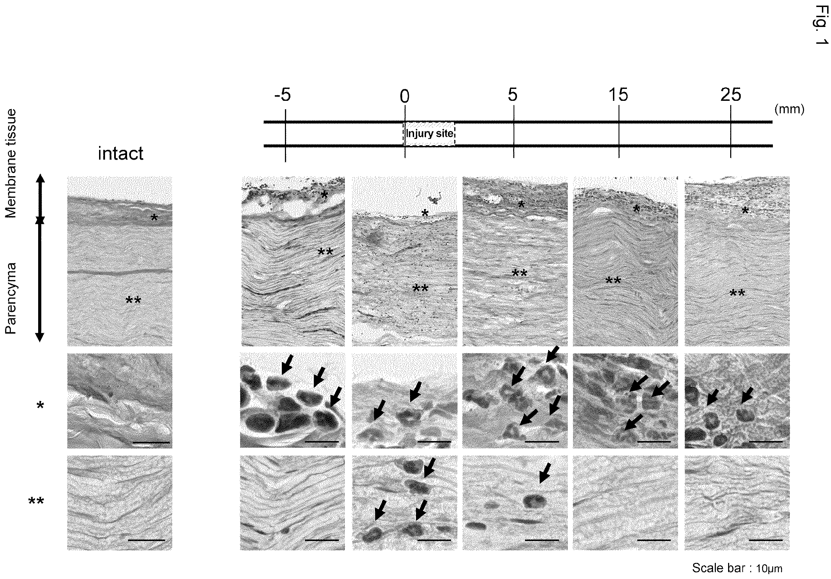

[FIG. 1] FIG. 1 includes representative HE-stained images of longitudinal sections of the rat sciatic nerve 12 hours after crush injury. The upper panels are low magnification images, the middle panels are high magnification images of the region corresponding to the epineurium (area marked with * in the low magnification images), and the lower panels are high magnification images of the region corresponding to the nerve parenchyma (area marked with ** in the low magnification images). Arrows indicate neutrophils.

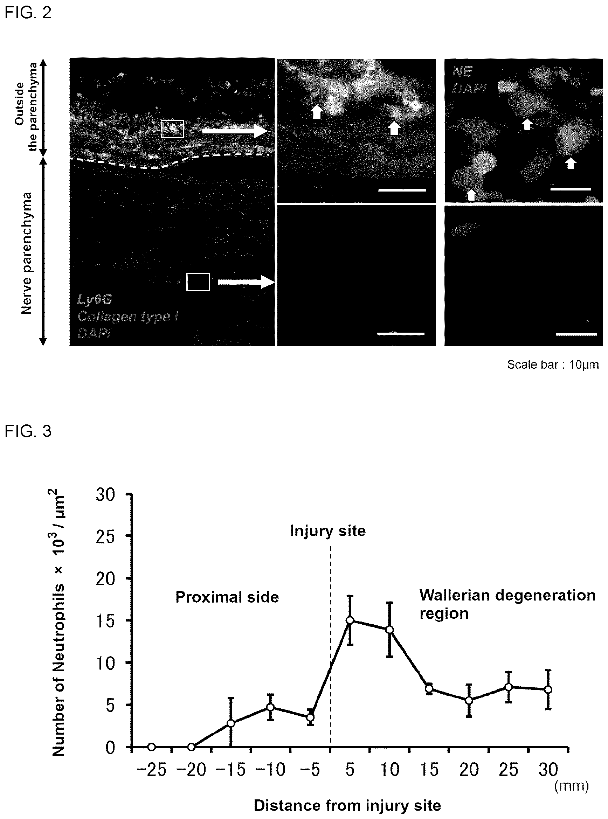

[FIG. 2] FIG. 2 includes representative immunostained images of a longitudinal section at 20 mm distal to the injury site of the rat sciatic nerve 12 hours after crush injury. The three images on the left and in the center are merged images of Ly-6G immunostaining, type I collagen immunostaining, and DAPI staining, and the left is a low magnification image, the upper center is a high magnification image of the region corresponding to the epineurium (upper boxed area in the left image), and the lower center is a high magnification image of the region corresponding to the nerve parenchyma (lower boxed area in the left image). The two images on the right are merged images of Neutrophil elastase (NE) immunostaining and DAPI staining, and the upper right is a high magnification image of the region corresponding to the epineurium, and the lower right is a high magnification image of the region corresponding to the nerve parenchyma. The dashed line in the image indicates the border between the parenchyma and outside the parenchyma (epineurium) of the nerve, and the arrows indicate neutrophils.
[FIG. 3] FIG. 3 is a graph of the number of neutrophils per unit area of nerve detected in the rat sciatic nerves 12 hours after crush injury, plotted against the distance from the injury site.

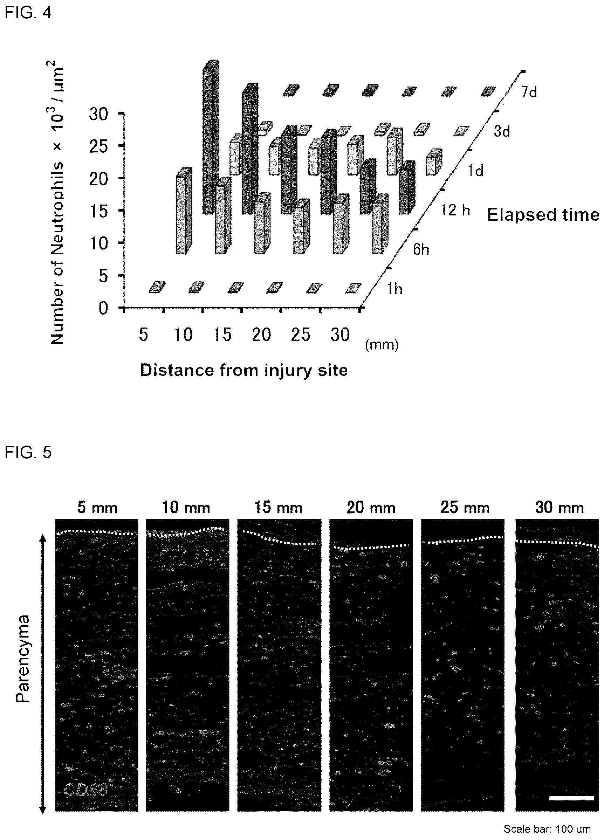

[FIG. 4] FIG. 4 is a graph of the number of neutrophils per unit area of nerve detected in the rat sciatic nerves after crush injury, by distance from the injury site and time elapsed since the injury was created.
[FIG. 5] FIG. 5 includes representative CD68 (a marker for macrophages) -immunostained images of longitudinal sections of the rat sciatic nerve 7 days after crush injury. The dashed lines in the images indicate the border between the parenchyma and outside the parenchyma (epineurium) of the nerve.

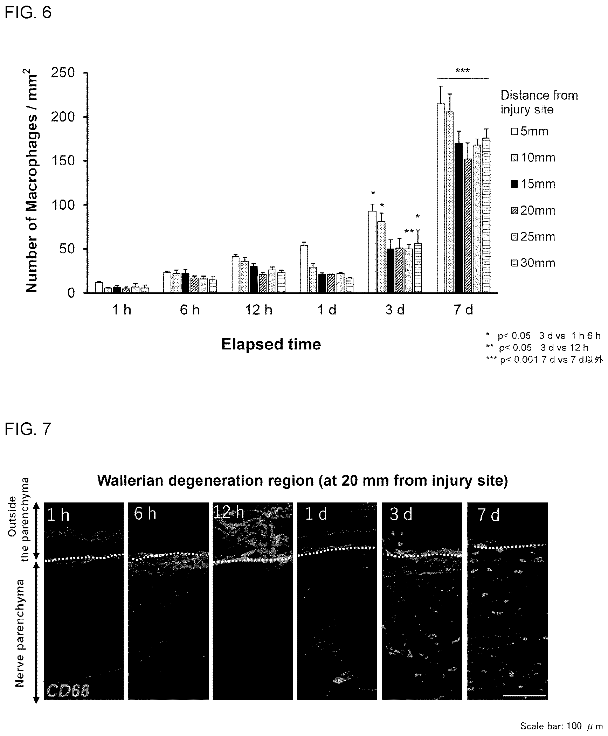

[FIG. 6] FIG. 6 is a graph of the number of macrophages per unit area of nerve parenchyma detected in the rat sciatic nerves after crush injury, by distance from the injury site and time elapsed since the injury was created.
[FIG. 7] FIG. 7 includes representative CD68-immunostained images of longitudinal sections at 20 mm distal to the injury site of the rat sciatic nerves from 1 hour to 7 days after crush injury. The dashed lines in the images indicate the border between the parenchyma and outside the parenchyma (epineurium) of the nerve.

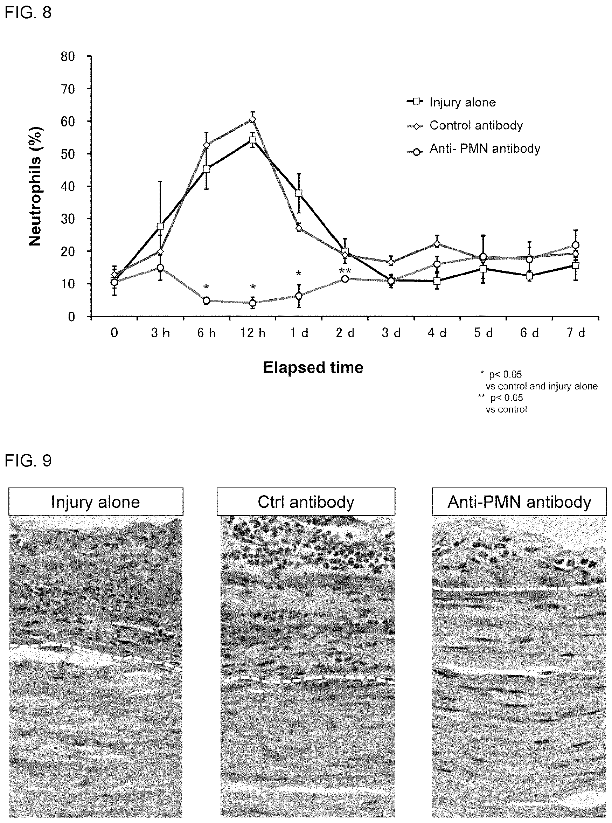

[FIG. 8] FIG. 8 is a graph illustrating the change over time in the number of neutrophils in the blood of rats administered an anti-neutrophil antibody or control antibody intraperitoneally immediately after crush injury (anti-PMN antibody and Ctrl antibody, respectively, in the figures) and rats that underwent only crush injury (injury alone, in the figures).
[FIG. 9] FIG. 9 includes representative HE-stained images of longitudinal sections at 5 mm distal to the injury site of the sciatic nerve in rats 12 hours after crush injury. The rats were administered an anti-neutrophil antibody or control antibody intraperitoneally immediately after crush injury, or underwent only crush injury.

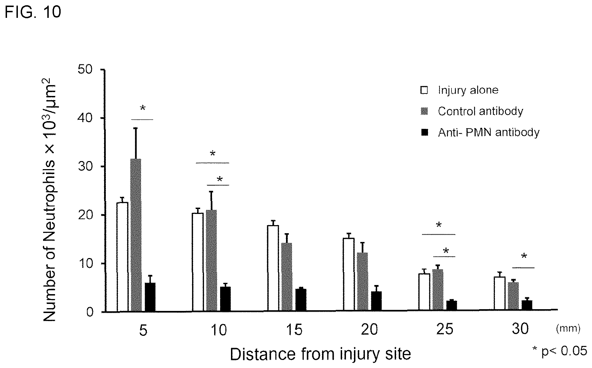

[FIG. 10] FIG. 10 is a graph of the number of neutrophils per unit area of nerve detected in the sciatic nerve in rats 12 hours after crush injury, by distance from the injury site. The rats were administered an anti-neutrophil antibody or control antibody intraperitoneally immediately after crush injury, or underwent only crush injury.

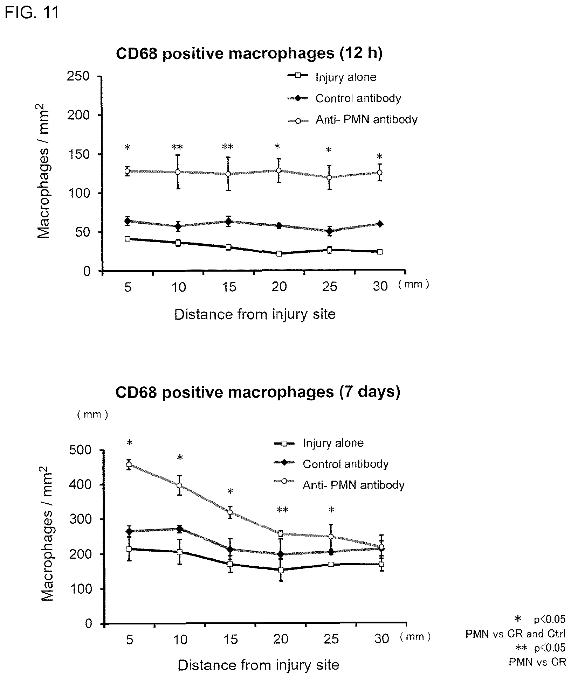

[FIG. 11] FIG. 11 includes graphs of the number of macrophages per unit area of nerve parenchyma detected in the sciatic nerve in rats 12 hours (upper panel) and 7 days (lower panel) after crush injury, plotted against the distance from the injury site. The rats were administered an anti-neutrophil antibody or control antibody intraperitoneally immediately after crush injury, or underwent only crush injury.

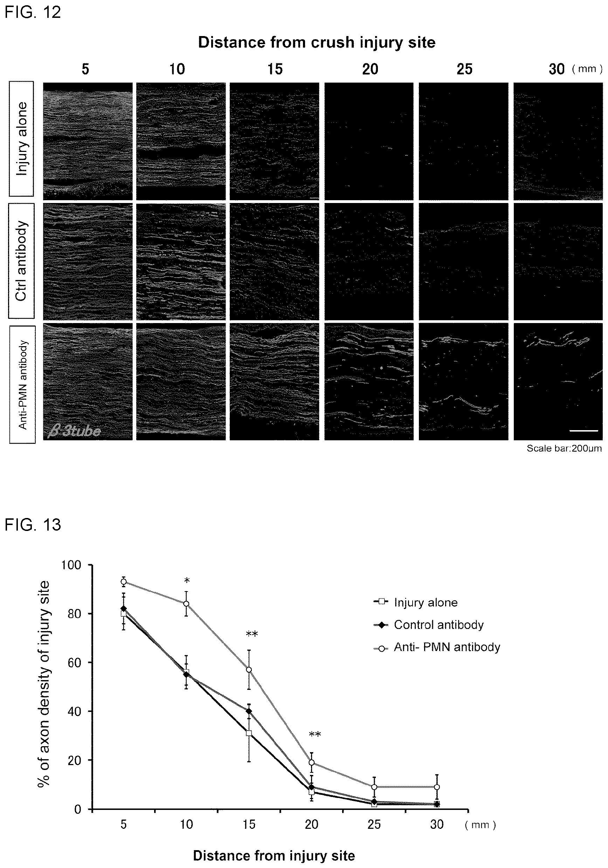

[FIG. 12] FIG. 12 includes representative β3 tubulin-immunostained images of longitudinal sections of the sciatic nerve in rats 7 days after crush injury. The rats were administered an anti-neutrophil antibody or control antibody intraperitoneally immediately after crush injury, or underwent only crush injury.
[FIG. 13] FIG. 13 is a graph of the axonal regeneration percentage of the sciatic nerve in rats 7 days after crush injury, plotted against the distance from the injury site. The rats were administered an anti-neutrophil antibody or control antibody intraperitoneally immediately after crush injury, or underwent only crush injury.

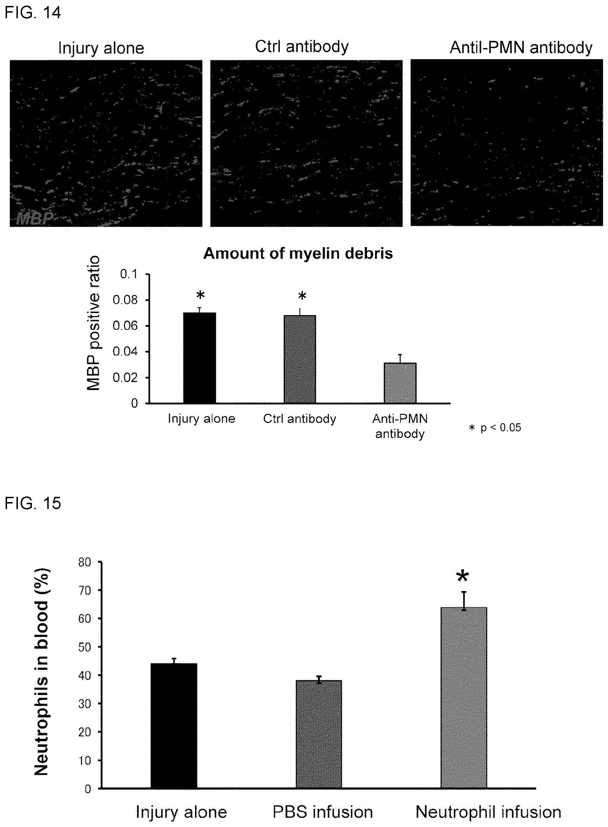

[FIG. 14] FIG. 14 includes representative MBP(Myelin Basic Protein)-immunostained images of longitudinal sections (at 25 mm distal to the injury site) of the sciatic nerve in rats 7 days after crush injury (upper panels), and a graph of the MBP positive ratio (lower panel). The rats were administered an anti-neutrophil antibody or control antibody intraperitoneally immediately after crush injury, or underwent only crush injury.
[FIG. 15] FIG. 15 is a graph illustrating the number of neutrophils 12 hours after crush injury in the blood of mice administered neutrophils or PBS intravenously (Neutrophil infusion and PBS infusion, respectively, in the figure), and mice that underwent only crush injury (injury alone, in the figure).

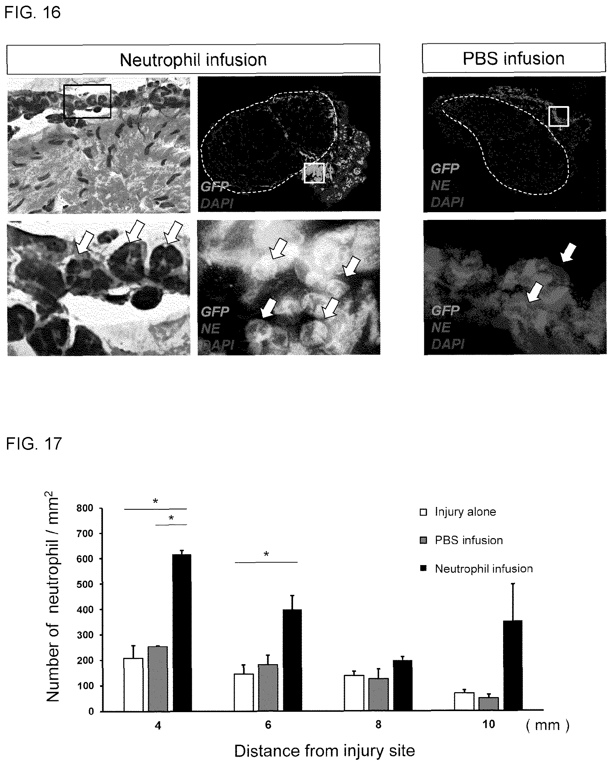

[FIG. 16] FIG. 16 includes representative HE-stained and immunostained images of transverse sections at 4 mm distal to the injury site of the sciatic nerve 12 hours after crush injury in mice administered neutrophils or PBS intravenously. The two images on the left are HE-stained images of mice administered neutrophils, the two images in the middle are immunostained images of mice administered neutrophils, and the two images on the right are immunostained images of mice administered PBS. All images in the lower panels are high magnification images of the region corresponding to the epineurium (areas within the box in the corresponding upper panel images). The dashed lines in the images indicate the border between the parenchyma and outside the parenchyma (epineurium) of the nerve, the regions surrounded by dashed lines represent the nerve parenchyma, and the arrows indicate neutrophils.
[FIG. 17] FIG. 17 is a graph of the number of neutrophils per unit area of nerve detected in the sciatic nerve 12 hours after crush injury in mice administered neutrophils or PBS intravenously and mice that underwent only crush injury, by distance from the injury site.

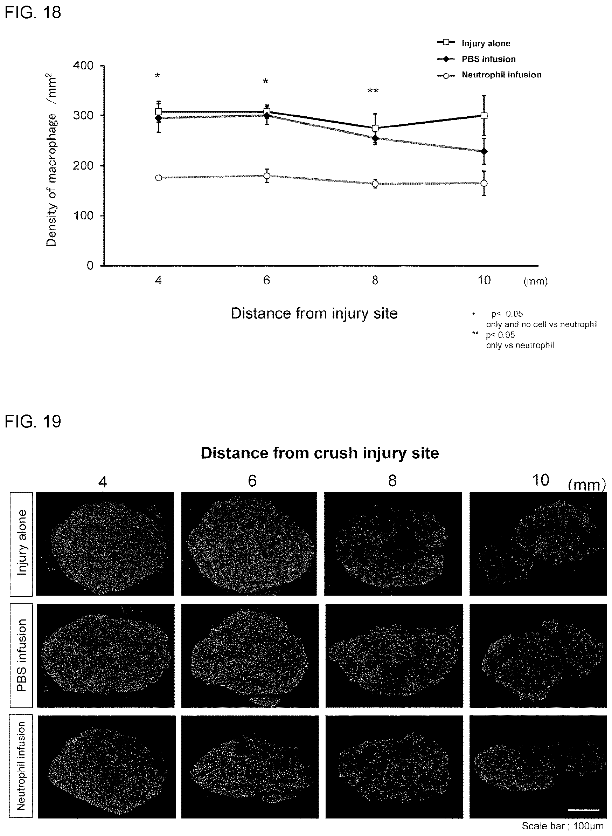

[FIG. 18] FIG. 18 is a graph of the number of macrophages per unit area of nerve parenchyma detected in the sciatic nerve 7 days after crush injury in mice administered neutrophils or PBS intravenously and mice that underwent only crush injury, plotted against the distance from the injury site.
[FIG. 19] FIG. 19 includes representative β3 tubulin-immunostained images of transverse sections of the sciatic nerve 7 days after crush injury in mice administered neutrophils or PBS intravenously and mice that underwent only crush injury.

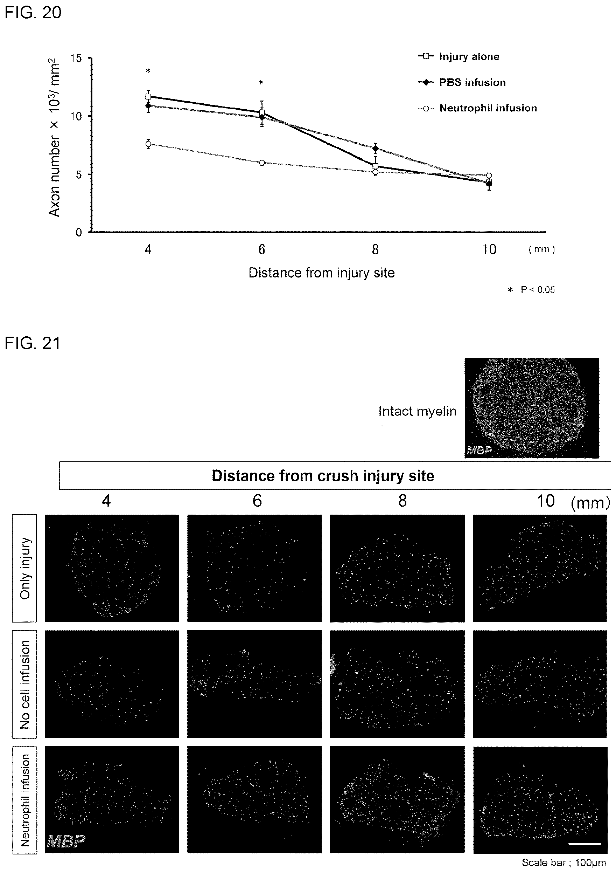

[FIG. 20] FIG. 20 is a graph of the axon density in the sciatic nerve 7 days after crush injury in mice administered neutrophils or PBS intravenously and mice that underwent only crush injury, plotted against the distance from the injury site.
[FIG. 21] FIG. 21 includes representative MBP-immunostained images of transverse sections of the sciatic nerve 7 days after crush injury in mice administered neutrophils or PBS intravenously and mice that underwent only crush injury.

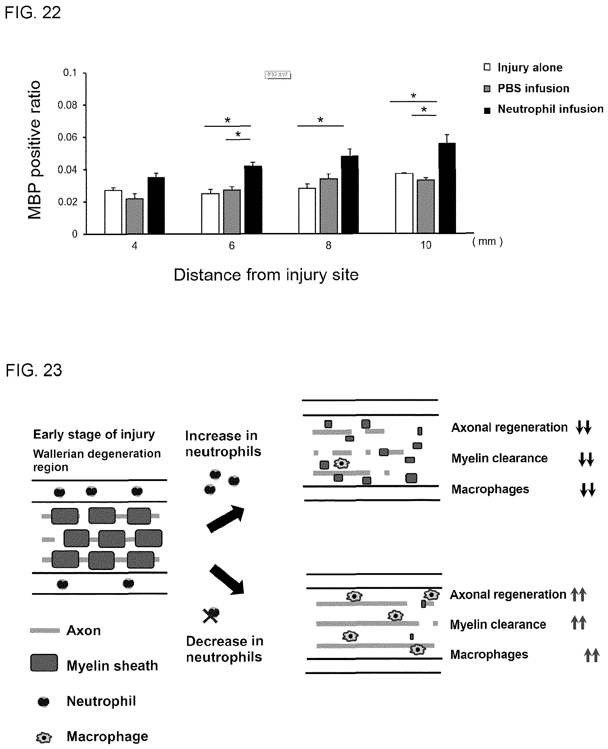

[FIG. 22] FIG. 22 is a graph of the MBP positive ratio of the sciatic nerve 7 days after crush injury in mice administered neutrophils or PBS intravenously and mice that underwent only crush injury, by distance from the injury site.
[FIG. 23] FIG. 23 is a conceptual diagram illustrating the process of axonal regeneration controlled by neutrophils.

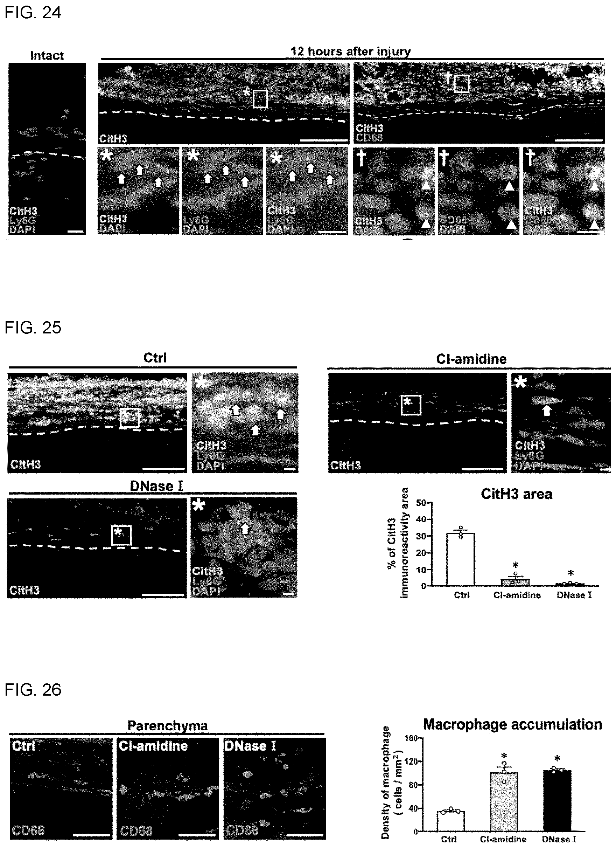

[FIG. 24] FIG. 24 includes representative immunostained images of a longitudinal section of the intact sciatic nerve in an untreated rat, and longitudinal sections at 20 mm distal to the injury site of the sciatic nerve in rats 12 hours after crush injury. The images are CitH3-immunostained, Ly6G-immunostained, CD68-immunostained, DAPI-stained, or merged images of several of these, respectively. The top center and top right images are low magnification images (scale bar 100 µm), and the three images (scale bar 10 µm) each in the bottom center and bottom right are high magnification images of the boxed areas. The dashed lines in the images indicate the border between the parenchyma and outside the parenchyma (epineurium) of the nerve, and the arrows indicate the co-localization of NETs and neutrophils.
[FIG. 25] FIG. 25 includes representative CitH3- and Ly-6G-immunostained images of longitudinal sections at 20 mm distal to the injury site of the sciatic nerve in rats 12 hours after crush injury, and a graph of the CitH3 positive area. The sciatic nerves were treated from 15 mm to 25 mm distal to the injury site with either a sheet containing the PAD4 inhibitor Cl-amidine, a sheet containing DNase I, or a control sheet. In each group, the image on the left is a low magnification image (scale bar 100 µm), and the image on the right is high magnification image of the boxed area (scale bar 10 µm). The dashed lines in the images indicate the border between the parenchyma and outside the parenchyma (epineurium) of the nerve.
[FIG. 26] FIG. 26 includes representative CD68-immunostained images (scale bar 50 µm) of longitudinal sections at 20 mm distal to the injury site of the sciatic nerve in rats 12 hours after crush injury, and a graph of the number of macrophages per unit area of nerve parenchyma. The sciatic nerves were treated from 15 mm to 25 mm distal to the injury site with either a sheet containing the PAD4 inhibitor Cl-amidine, a sheet containing DNase I, or a control sheet.

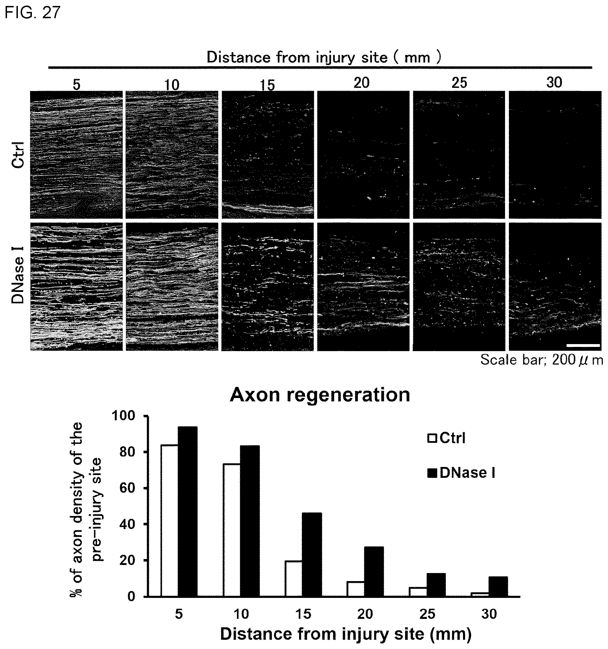

[FIG. 27] FIG. 27 includes representative β3 tubulin-immunostained images of longitudinal sections of the sciatic nerve in rats 7 days after crush injury (upper panels), and a graph of the axonal regeneration percentage of the sciatic nerves by distance from the injury site (lower panel). The sciatic nerves were treated from 5 mm to 25 mm distal to the injury site with a sheet containing DNase I.

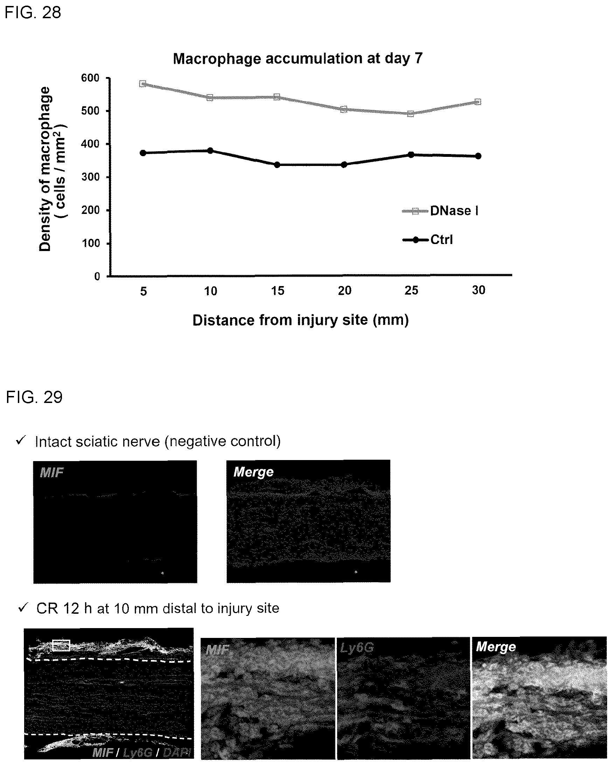

[FIG. 28] FIG. 28 is a graph of the number of macrophages per unit area of nerve parenchyma detected in the sciatic nerve in rats 7 days after crush injury, plotted against the distance from the injury site. The sciatic nerves were treated from 5 mm to 25 mm distal to the injury site with a sheet containing DNase I.
[FIG. 29] FIG. 29 includes representative MIF-immunostained images of a longitudinal section of the intact sciatic nerve in an untreated rat (upper panels), and longitudinal sections at 10 mm distal to the injury site of the sciatic nerve in rats 12 hours after crush injury (lower panels). The two images in the upper panels and the left image in the lower panels are low magnification images, and the three images on the right in the lower panels are high magnification images of the region corresponding to the epineurium indicated by the boxed area in the left image in the lower panels. The image with "Merge" in the lower right is a merged image of MIF immunostaining, Ly-6G immunostaining, and DAPI staining. The dashed lines in the images indicate the border between the parenchyma and outside the parenchyma (epineurium) of the nerve.

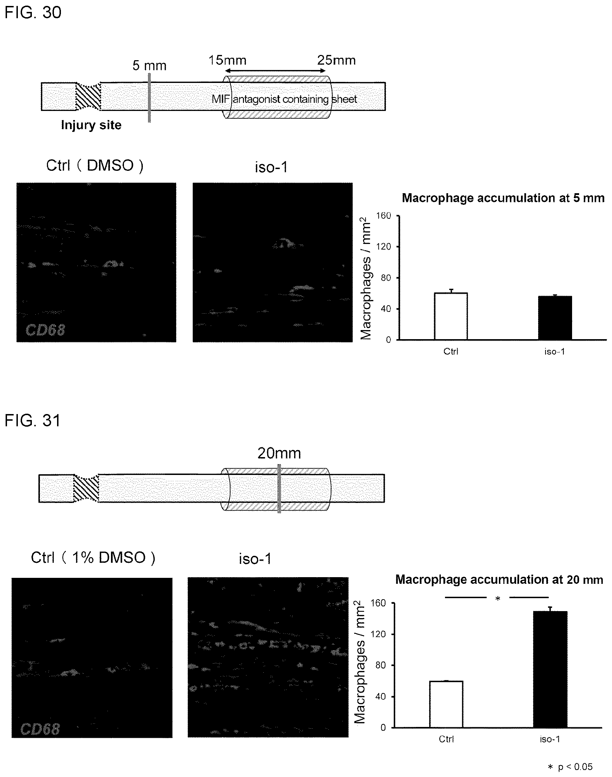

[FIG. 30] FIG. 30 includes representative CD68-immunostained images of longitudinal sections at 5 mm distal to the injury site of the sciatic nerve in rats 12 hours after crush injury, and a graph of the number of macrophages per unit area of nerve parenchyma. The sciatic nerves were treated from 15 mm to 25 mm distal to the injury site with either a sheet containing the MIF inhibitor iso-1, or a control sheet.
[FIG. 31] FIG. 31 includes representative CD68-immunostained images of longitudinal sections at 20 mm distal to the injury site of the sciatic nerve in rats 12 hours after crush injury, and a graph of the number of macrophages per unit area of nerve parenchyma. The sciatic nerves were treated from 15 mm to 25 mm distal to the injury site with either a sheet containing iso-1, or a control sheet.

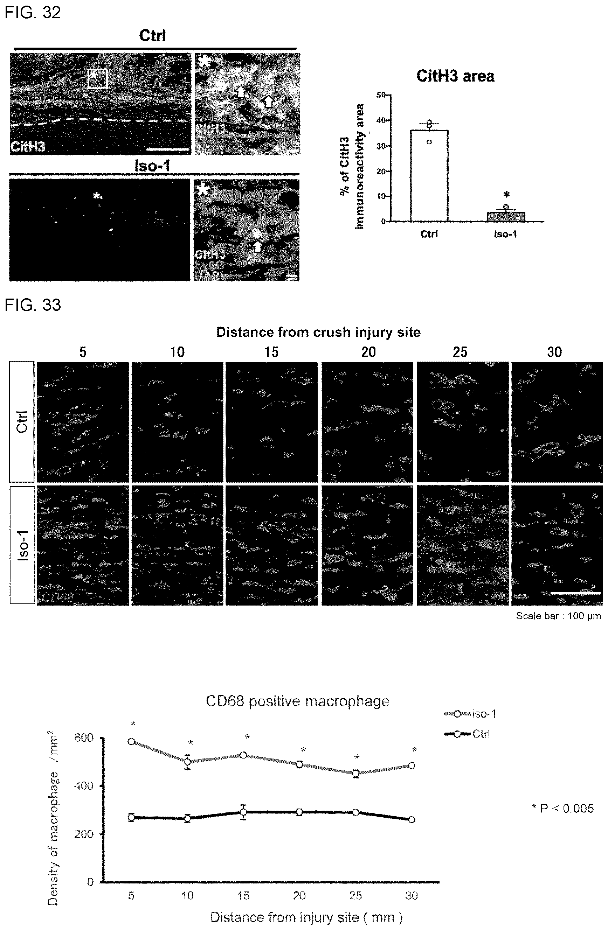

[FIG. 32] FIG. 32 includes representative immunostained images of longitudinal sections at 20 mm distal to the injury site of the sciatic nerve in rats 12 hours after crush injury, and a graph of the CitH3 positive area. The sciatic nerves were treated from 15 mm to 25 mm distal to the injury site with either a sheet containing iso-1, or a control sheet. In each group, the image on the left is a low magnification image (scale bar 100 µm), and the image on the right is high magnification image of the boxed area (scale bar 10 µm). The dashed line in the images indicates the border between the parenchyma and outside the parenchyma (epineurium) of the nerve.
[FIG. 33] FIG. 33 includes representative CD68-immunostained images of longitudinal sections of the sciatic nerve in rats 7 days after crush injury, and a graph of the number of macrophages per unit area of nerve parenchyma. The sciatic nerves were treated from 5 mm to 25 mm distal to the injury site with either a sheet containing iso-1, or a control sheet.

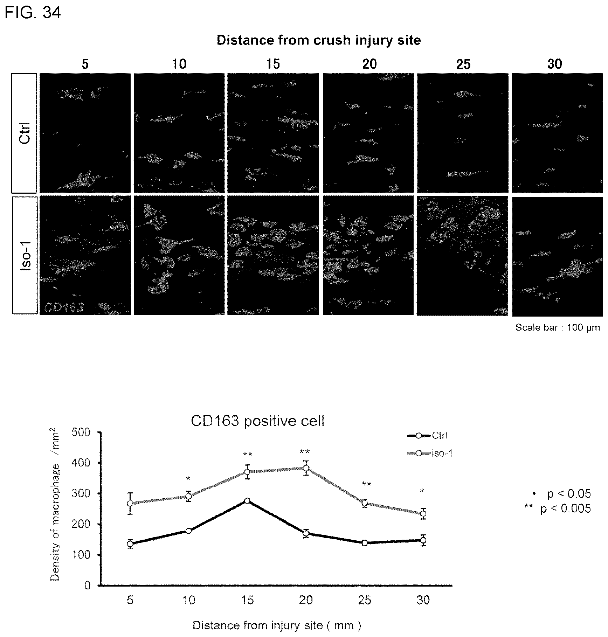

[FIG. 34] FIG. 34 includes representative CD163-immunostained images of longitudinal sections of the sciatic nerve in rats 7 days after crush injury, and a graph of the number of M2 macrophages per unit area of nerve parenchyma. The sciatic nerves were treated from 5 mm to 25 mm distal to the injury site with either a sheet containing iso-1, or a control sheet.

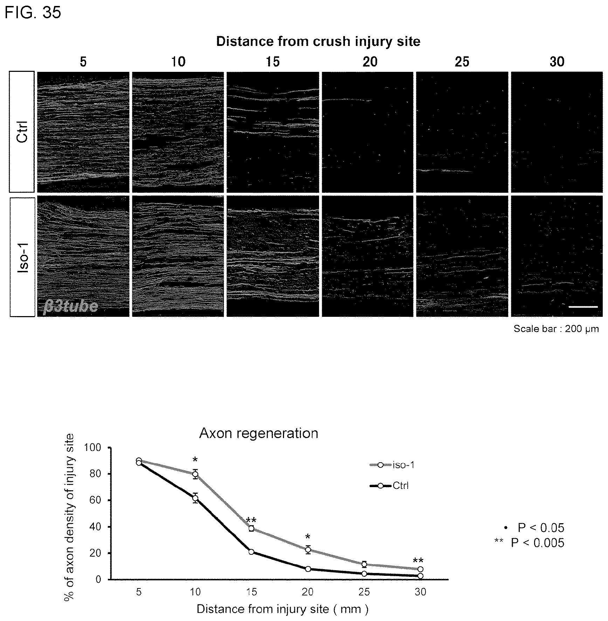

[FIG. 35] FIG. 35 includes representative β3 tubulin-immunostained images of longitudinal sections of the sciatic nerve in rats 7 days after crush injury, and a graph of the axonal regeneration percentage of the sciatic nerves plotted against the distance from the injury site. The sciatic nerves were treated from 5 mm to 25 mm distal to the injury site with either a sheet containing iso-1, or a control sheet.

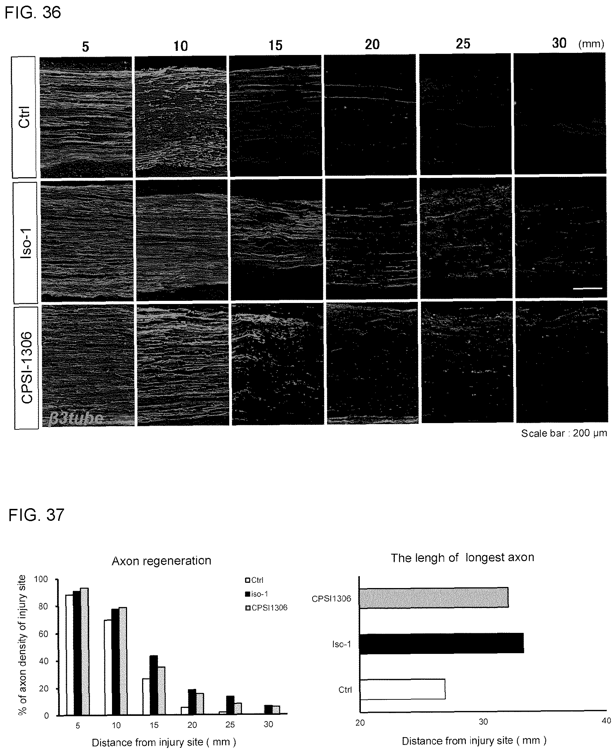

[FIG. 36] FIG. 36 includes representative β3 tubulin-immunostained images of longitudinal sections of the sciatic nerve in rats 7 days after crush injury. The sciatic nerves were treated from 5 mm to 25 mm distal to the injury site with either a sheet containing iso-1, a sheet containing the MIF inhibitor CPSI-1306, or a control sheet.
[FIG. 37] FIG. 37 includes graphs of the axonal regeneration percentage plotted against the distance from the injury site (left panel) and the longest axon length (right panel) of the sciatic nerve in rats 7 days after crush injury. The sciatic nerves were treated from 5 mm to 25 mm distal to the injury site with either a sheet containing iso-1, a sheet containing the MIF inhibitor CPSI-1306, or a control sheet.

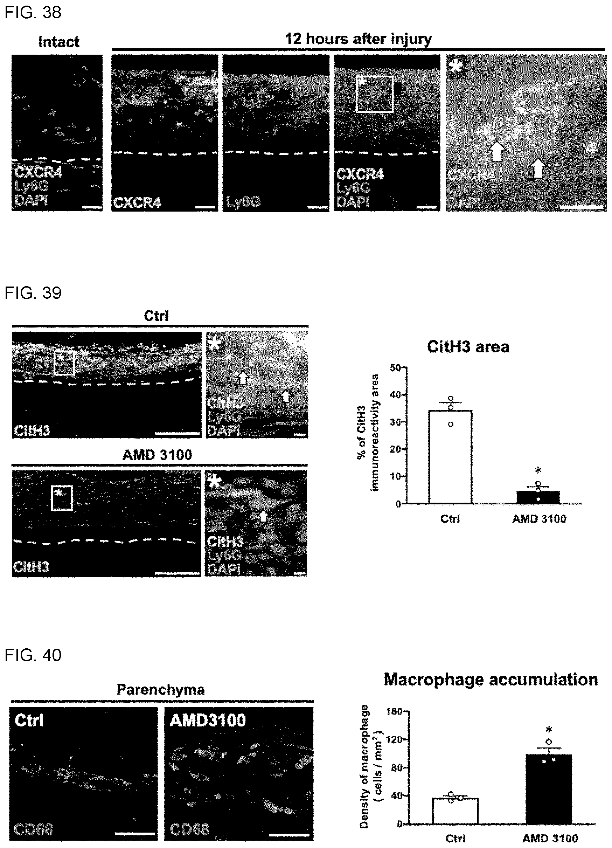

[FIG. 38] FIG. 38 includes representative immunostained images of a longitudinal section of the intact sciatic nerve in an untreated rat, and longitudinal sections at 20 mm distal to the injury site of the sciatic nerve in rats 12 hours after crush injury. The second image from the left shows CXCR4 immunostaining, the third image from the left shows Ly-6G immunostaining, and the other images are merged images of CitH3 immunostaining, Ly-6G immunostaining, and DAPI staining. The four images on the left are low magnification images (scale bar 20 µm), and the image on the right is a high magnification images of the boxed area. The dashed lines in the images indicate the border between the parenchyma and outside the parenchyma (epineurium) of the nerve, and the arrows indicate the co-localization of CXCR4 and neutrophils.
[FIG. 39] FIG. 39 includes representative immunostained images of longitudinal sections at 20 mm distal to the injury site of the sciatic nerve in rats 12 hours after crush injury, and a graph of the CitH3 positive area. The sciatic nerves were treated from 15 mm to 25 mm distal to the injury site with either a sheet containing AMD3100, or a control sheet. In each group, the image on the left is a low magnification image (scale bar 100 µm), and the image on the right is high magnification image of the boxed area (scale bar 10 µm). The dashed lines in the images indicate the border between the parenchyma and outside the parenchyma (epineurium) of the nerve.
[FIG. 40] FIG. 40 includes representative CD68-immunostained images (scale bar 50 µm) of longitudinal sections at 20 mm distal to the injury site of the sciatic nerve in rats 12 hours after crush injury, and a graph of the number of macrophages per unit area of nerve parenchyma. The sciatic nerves were treated from 15 mm to 25 mm distal to the injury site with either a sheet containing AMD3100, or a control sheet.

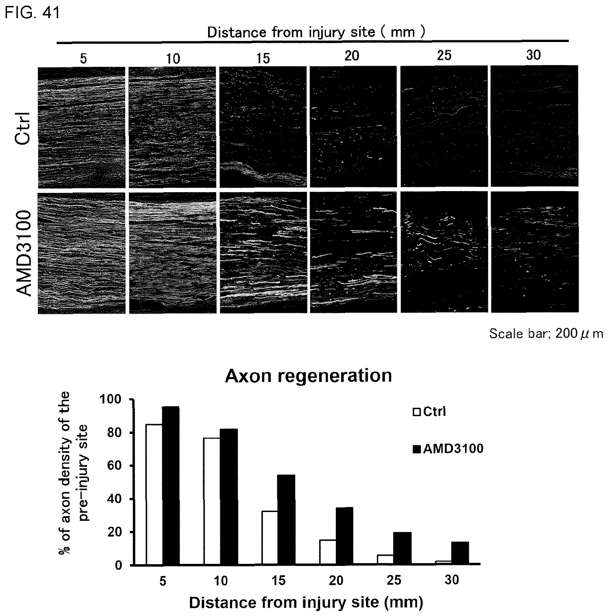

[FIG. 41] FIG. 41 includes representative β3 tubulin-immunostained images of longitudinal sections of the sciatic nerve in rats 7 days after crush injury (upper panels), and a graph of the axonal regeneration percentage of the sciatic nerves by distance from the injury site (lower panel). The sciatic nerves were treated from 5 mm to 25 mm distal to the injury site with either a sheet containing AMD3100, or a control sheet.

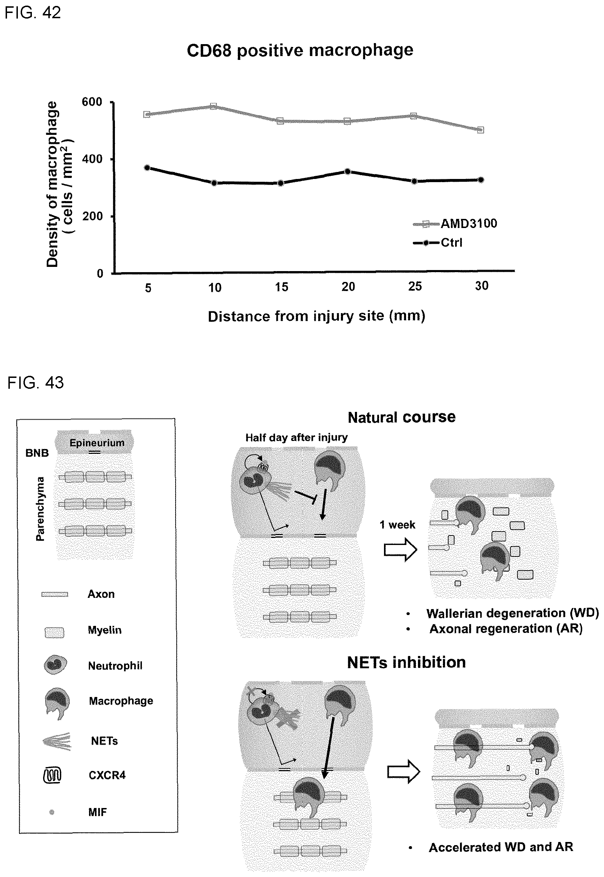

[FIG. 42] FIG. 42 is a graph of the number of macrophages per unit area of nerve parenchyma detected in the sciatic nerve in rats 7 days after crush injury, plotted against the distance from the injury site. The sciatic nerves were treated from 5 mm to 25 mm distal to the injury site with a sheet containing AMD3100.
[FIG. 43] FIG. 43 is a conceptual diagram illustrating the process of axonal regeneration controlled via NETs by neutrophils.

### Description of Embodiments

The following description may be based on representative embodiments or specific examples, but the present invention is not limited to such embodiments or specific examples. The description of proteins and genes will be given with human-derived proteins and genes as examples unless otherwise specified, but proteins and genes used in the present invention are not limited to human-derived ones, and the subject to which the present invention is applied is not limited to humans.

In the present specification, the upper limit value and the lower limit value of each numerical range can be combined as desired. In the present specification, a numerical range represented using "to" or "-" means a range including numerical values at both ends thereof as an upper limit value and a lower limit value, unless otherwise noted.

In one aspect, the present invention provides a pharmaceutical composition for treating peripheral nerve injury, containing a substance that suppresses the formation of NETs outside the parenchyma of an injured peripheral nerve.

Neutrophils are a type of granulocyte that, after differentiation and maturation in the bone marrow, are normally stored in the bone marrow (reservoir pool), peripheral blood (circulating pool), and vascular walls and tissues (marginal pool). When inflammation occurs due to tissue injury, neutrophils accumulate at the injury site in response to neutrophil migration stimulating factors such as inflammatory cytokines and chemokines produced at the inflammatory site, and are known to be involved in tissue repair and regeneration.

NETs are reticular structures composed of chromatin, containing citrullinated histone proteins, and intracellular antimicrobial proteins, and are released by neutrophils. NETs are known to be involved in host defense and autoimmune diseases (Kely Campos Navegantes et al., Journal of Translational Medicine, 2017, vol. 15, no. 36, DOI 10.1186/s12967-017-1141-8).

The inventors of the present invention have found that in the distal regions to the injury site of peripheral nerves, neutrophils first accumulate outside the nerve parenchyma, and then macrophages accumulate in the nerve parenchyma with the disappearance of neutrophils, that inhibition of neutrophil accumulation causes macrophages to accumulate early and in large numbers in the nerve parenchyma, leading to the promotion of axonal regeneration and myelin clearance, and that, in contrast, an increase in the number of neutrophils accumulated suppresses macrophage accumulation, and axonal regeneration and myelin clearance are also suppressed.

The inventors have further found that in the distal regions to the injury site of peripheral nerves, NETs are located outside the nerve parenchyma and overlap their localization with neutrophils, and that suppression of NETs formation outside the nerve parenchyma causes macrophages to accumulate early and in large numbers in the nerve parenchyma, leading to the promotion of axonal regeneration. A substance that suppresses the formation of NETs is useful for the treatment of peripheral nerve injury.

The parenchyma of a peripheral nerve means the internal region of the peripheral nerve surrounded by the epineurium, and the parenchyma includes the nerve bundle in which axons are wrapped by the perineurium. Outside the parenchyma of the nerve means the epineurium and the surrounding area, particularly the epineurium. A substance that suppresses the formation of NETs outside the parenchyma of an injured peripheral nerve includes a substance that inhibits the accumulation of neutrophils in an injured peripheral nerve, a substance that inhibits NETs formation by neutrophils, and a substance that degrades NETs.

### Substance that inhibits accumulation of neutrophils in injured peripheral nerve

The substance that inhibits the accumulation of neutrophils in an injured peripheral nerve used in the present disclosure is any substance having the ability to inhibit the accumulation of neutrophils in an injured peripheral nerve, and is preferably at least one substance selected from the group consisting of an antibody against neutrophils and antigen-binding fragments thereof, an aptamer targeting neutrophils, an inhibitor of neutrophil migration stimulating factor, and a neutrophil migration inhibitory factor.

One example of the substance that inhibits the accumulation of neutrophils in an injured peripheral nerve is an antibody against neutrophils or an antigen-binding fragment thereof. The antibody against neutrophils can be any antibody that can bind to neutrophils, for example, to a protein on the cell surface of neutrophils, thereby reducing the number of neutrophils or inhibiting neutrophil function, and is preferably a specific antibody against neutrophils. A specific antibody against neutrophils includes, for example, an antibody against Ly-6G (also called myeloid cell differentiation antigen Gr-1), which is a GPI-anchored protein of the Ly-6 family, an anti-PMN (polymorphonuclear neutrophil) antibody, an anti-RP3 antibody.

In the present disclosure, a specific antibody can also be represented as an antibody that binds preferentially to a target protein over non-target proteins, or an antibody having high binding affinity for a target protein. The specific antibody can bind to the target protein with affinity at least 5 times stronger, preferably at least 10 times stronger, more preferably at least 100 times stronger, and most preferably at least 1000 times stronger than affinity to the non-target proteins. The specific antibody can also be an antibody that binds to the target protein with a dissociation constant of 10⁻⁷ M, preferably 10⁻⁸ M, more preferably 10⁻⁹ M.

The antibody used in the present disclosure can originate from any species including, for example, mice, rats, sharks, rabbits, pigs, hamsters, camels, llamas, goats, or humans. The antibody can be of any class (for example, IgG, IgE, IgM, IgD, or IgA) and subclass of immunoglobulin molecules, and IgG is preferred.

The antibody used in the present disclosure can be a polyclonal antibody or a monoclonal antibody, and a monoclonal antibody is preferred. The antibody may be a chimeric antibody, a humanized antibody, or a human antibody.

In the present disclosure, an antigen-binding fragment of an antibody is defined as a partial fragment of the antibody having the ability to bind to the target protein of the antibody. The antigen-binding fragment of an antibody is preferably a partial fragment of a specific antibody having the ability to bind specifically to the target protein of the antibody, and can include, but not limited to, a fragment of antigen binding (Fab), Fab', F(ab')2, single chain Fv, disulfide stabilized Fv, and a peptide containing the CDRs of the antibody.

The antibody and antigen-binding fragment thereof can be produced using methods known to those skilled in the art. For example, they can be produced by preparing the target protein by genetic recombination techniques based on the amino acid sequence or the nucleotide sequence of DNA encoding the target protein, immunizing an appropriate animal with the target protein as an antigen, and then fusing B cells of the animal with myeloma cells to obtain hybridomas. For the hybridoma method, see, for example, Meyaard et al. (1997) Immunity 7:283-290; Wright et al. (2000) Immunity 13:233-242; Kaithamana et al. (1999) J.Immunol. 163:5157-5164. The antibody and antigen-binding fragment thereof may be further conjugated with other drugs to form antibody-drug conjugates.

Another example of the substance that inhibits the accumulation of neutrophils in an injured peripheral nerve is an aptamer targeting neutrophils. The aptamer targeting neutrophils can bind to neutrophils, for example, to a protein on the cell surface of neutrophils, thereby reducing the number of neutrophils or inhibiting neutrophil function. One example of the aptamer targeting neutrophils is an aptamer targeting Ly-6G.

An aptamer is a molecule that has the ability to bind specifically to a target substance by its three-dimensional structure. An aptamer composed of nucleic acids is called a nucleic acid aptamer, and an aptamer composed of amino acids is called a peptide aptamer. In the present disclosure, both of these aptamers, the nucleic acid aptamer and the peptide aptamer, can be used.

The nucleic acid aptamer used in the present disclosure can be deoxyribonucleic acid (DNA), ribonucleic acid (RNA), or a chimeric nucleic acid containing both deoxyribonucleotides and ribonucleotides as its component units. The nucleic acid aptamer can be produced by methods known to those skilled in the art, such as the SELEX (systematic evolution of ligands by exponential enrichment) method. The peptide aptamer can be produced by methods known to those skilled in the art, such as the phage display method and cell surface display method.

Another example of the substance that inhibits the accumulation of neutrophils in an injured peripheral nerve is a substance that exhibits inhibitory activity against a factor that promotes neutrophil migration (also called neutrophil migration stimulating factor, neutrophil migration factor, or neutrophil migration activating factor), examples of the factor include cytokines such as interleukin 8 (IL-8), IL-17, growth-related gene products alpha (Gro alpha), neutrophil activating protein-2 (NAP-2), macrophage inflammatory protein 2 (MIP-2); lipid mediators such as leukotriene B4; N-formyl-methionyl-leucyl-phenylanine (fMLP); and complement components such as C5a. The substance that exhibits inhibitory activity against the factor is referred to herein as an inhibitor of neutrophil migration stimulating factor. Examples of such an inhibitor include an antibody against neutrophil migration stimulating factor, an antagonist for the receptor of neutrophil migration stimulating factor.

Another example of the substance that inhibits the accumulation of neutrophils in an injured peripheral nerve is a factor that inhibits neutrophil migration (also called neutrophil migration inhibitory factor) such as colchicine, resolvin, protectin, and 17β estradiol.

### Substance that inhibits NETs formation by neutrophils

NETs are formed in the cytoplasm of neutrophils through citrullination of histone proteins by peptidyl arginine deiminase 4 (PAD4) in the nucleus, decondensation of chromatin, influx of nuclear chromatin into the cytoplasm following nuclear membrane rupture, and subsequent binding of the chromatin to antibacterial proteins in granules. NETs are released extracellularly upon plasma membrane disruption of neutrophils. Neutrophils that release NETs undergo cell death called NETosis.

The substance that inhibits NETs formation by neutrophils can be selected from the group consisting of a substance that suppresses the expression or inhibits the function of MIF, a substance that suppresses the expression or inhibits the function of CXCR4, and a substance that suppresses the expression or inhibits the function of PAD4. That is, the substance that inhibits NETs formation by neutrophils can be selected from the group consisting of a substance that suppresses the expression of MIF, a substance that inhibits the function of MIF, a substance that suppresses the expression of CXCR4, a substance that inhibits the function of CXCR4, a substance that suppresses the expression of PAD4, and a substance that inhibits the function of PAD4.

MIF (glycosylation-inhibiting factor; also called GIF, L-dopachrome isomerase, or phenylpyruvate tautomerase) is a member of proinflammatory cytokines, and is expressed by many cells, including immune cells such as T cells, monocytes, neutrophils, macrophages, as well as pituitary cells and epithelial cells. MIF is known to be involved in both innate and acquired immunity and to induce the production of inflammatory molecules. The nucleotide sequence of human MIF gene is registered in NCBI Reference Sequence as NC_000022.11, the mRNA sequence as NM_002415.2, and the amino acid sequence as NP_002406.1 (both searched on July 19, 2021).

The inventors have found that 12 hours after injury, in the distal regions to the injury site of peripheral nerves, MIF is present only outside the nerve parenchyma and overlaps its localization with neutrophils, that inhibition of MIF inhibits the formation of NETs by neutrophils outside the parenchyma of the injured peripheral nerve, and that inhibition of MIF causes macrophages to accumulate early and in large numbers in the nerve parenchyma, leading to the promotion of axonal regeneration. Based on these findings, the present invention, in one aspect, utilizes a substance that suppresses the expression or inhibits the function of MIF in a pharmaceutical composition for treating peripheral nerve injury.

The substance that suppresses the expression or inhibits the function of MIF used in the present disclosure is any substance having the ability to suppress the expression of MIF at the gene or protein level or having the ability to inhibit the function of MIF, and is preferably at least one substance selected from the group consisting of a nucleic acid that suppresses the expression of MIF, an antibody against MIF and antigen-binding fragment thereof, an aptamer targeting MIF, and a MIF inhibitor.

One example of the substance that suppresses the expression of MIF is a nucleic acid that suppresses the expression of MIF. The nucleic acid that suppresses the expression of MIF encompasses a nucleic acid having the ability to suppress transcription of mRNA from MIF gene (MIF mRNA), a nucleic acid having the ability to degrade MIF mRNA, and a nucleic acid having the ability to suppress protein translation from MIF mRNA. Examples include an antisense nucleic acid or a nucleic acid causing RNA interference, such as siRNA, shRNA, or miRNA, which can be designed and produced by those skilled in the art based on the nucleotide sequence of MIF gene or the nucleotide sequence of MIF mRNA.

One example of the substance that inhibits the function of MIF is an antibody against MIF, preferably a specific antibody against MIF, or an antigen-binding fragment of an antibody against MIF. Another example of the substance that inhibits the function of MIF is an aptamer targeting MIF. Definitions and other descriptions of antibodies, specific antibodies, antigen-binding fragments, and aptamers are as described above.

Another example of the substance that inhibits the function of MIF is a MIF inhibitor, which is any substance that has the ability to inhibit the function of MIF. Examples of MIF inhibitor can include ISO-1 (CAS No. 478336-92-4; (S,R)-3-(4-Hydroxyphenyl)-4,5-dihydro-5-isoxazole acetic acid), (±)-CPSI 1306 (CAS No. 1309793-47-2; 2-[3-(3-(2,4-Difluorophenyl)-4,5-dihydro-5-isoxazolyl]-1-(4-morpholinyl)ethanone), 4-IPP (CAS No. 41270-96-6; 4-lodo-6-phenylpyrimidine), Ibudilast (CAS No. 50847-11-5; 1-[2-(1-Methylethyl)pyrazolo[1,5-a]pyridin-3-yl]-2-methylpropan-1-one), BTZO-1 (CAS No. 99420-15-2; 2-Pyridin-2-yl-4H-1,3-benzothiazin-4-one), Chicago Sky Blue 6B (CAS No. 2610-05-1; 6,6'-[(3,3-Dimethoxy[1,1'-biphenyl]-4,4'-diyl)bis(azo)]bis[4-amino-5-hydroxy-1,3-napthalenedisulphonic acid] tetrasodium salt), p425 (CAS No. 259444; Tetrasodium 6,6'-[(3,3'-dimethoxy[1,1'-biphenyl]-4,4'-diyl)bis(azo)]bis[4-amino-5-hydroxynaphthalene-1,3- disulphonate]).

In a preferred embodiment, the substance that inhibits the expression or function of MIF is a MIF inhibitor, in particular ISO-1 or (±)-CPSI 1306.

CXCR4 is a G protein-coupled receptor belonging to the CXC-motif chemokine receptor family and is known to induce migration of immune cells with its ligands CXC-motif chemokine ligand 12 (CXCL12) and stromal cell-derived factor 1 (SDF-1). The nucleotide sequence of human CXCR4 gene is registered in NCBI Reference Sequence as NC_000002.12, the mRNA sequences as NM_001008540.2, NM_001348056.2, NM_001348059.2, NM_001348060.2, NM_003467.3, and the amino acid sequences as NP_001008540.1, NP_001334985.1, NP_001334988.1, NP_001334989.1, and NP_003458.1 (all searched December 20, 2021).

The inventors have found that in the distal regions to the injury site of peripheral nerves, CXCR4 is present outside the nerve parenchyma and overlap its localization with neutrophils, and that inhibition of CXCR4 inhibits the formation of NETs by neutrophils outside the nerve parenchyma, and that inhibition of CXCR4 causes macrophages to accumulate early and in large numbers in the nerve parenchyma, leading to the promotion of axonal regeneration. A substance that suppresses the expression or inhibits the function of CXCR4 is useful in a pharmaceutical composition for treating peripheral nerve injury.

The substance that suppresses the expression or inhibits the function of CXCR4 is any substance having the ability to suppress the expression of CXCR4 at the gene or protein level or having the ability to inhibit the function of CXCR4, and is preferably at least one substance selected from the group consisting of a nucleic acid that suppresses the expression of CXCR4, an antibody against CXCR4 and antigen-binding fragment thereof, an aptamer targeting CXCR4, and a CXCR4 inhibitor.

One example of the substance that suppresses the expression of CXCR4 is a nucleic acid that suppresses the expression of CXCR4. The nucleic acid that suppresses the expression of CXCR4 encompasses a nucleic acid having the ability to suppress transcription of mRNA from CXCR4 gene (CXCR4 mRNA), a nucleic acid having the ability to degrade CXCR4 mRNA, and a nucleic acid having the ability to suppress protein translation from CXCR4 mRNA. Examples include an antisense nucleic acid or a nucleic acid causing RNA interference, such as siRNA, shRNA, or miRNA, which can be designed and produced by those skilled in the art based on the nucleotide sequence of CXCR4 gene or the nucleotide sequence of CXCR4 mRNA.

One example of the substance that inhibits the function of CXCR4 is an antibody against CXCR4, preferably a specific antibody against CXCR4, or an antigen-binding fragment of an antibody against CXCR4. Another example of the substance that inhibits the function of CXCR4 is an aptamer targeting CXCR4. Definitions and other descriptions of antibodies, specific antibodies, antigen-binding fragments, and aptamers are as described above.

Another example of the substance that inhibits the function of CXCR4 is a CXCR4 inhibitor, which is any substance that has the ability to inhibit the function of CXCR4. Examples of CXCR4 inhibitor can include Plerixafor (also called AMD3100; the general formula is 1,4-Bis[(1,4,8,11-tetraazacyclotetradecan-1-yl)methyl]benzene), WZ811 (CAS No. 55778-02-4; the general formula is 1,4-Benzenedimethanamine, N1,N4-di-2-pyridinyl-), Motixafortide (BL-8040, CAS No. 664334-36-5; the general formula is L-Argininamide, N2-(4-fluorobenzoyl)-L-arginyl-L-arginyl-3-(2-naphthalenyl)-L-alanyl-L-cysteinyl-L-tyrosyl-N5-(aminocarbonyl)-L-ornithyl-L-lysyl-D-lysyl-L-prolyl-L-tyrosyl-L-arginyl-N5-(aminocarbonyl)-L-ornithyl-L-cysteinyl-, cyclic (4-13)-disulfide).

PAD4 is an enzyme that induces NETs formation by converting specific arginine residues of histone proteins (H3, H4, etc.) to citrulline residues. The nucleotide sequence of human PAD4 gene is registered in NCBI Reference Sequence as NC_000001.11, the mRNA sequence as NM_012387.3, and the amino acid sequence as NP_036519.2 (both searched on December 20, 2021).

The inventors have found that inhibition of PAD4 inhibits the formation of NETs by neutrophils outside the parenchyma of an injured peripheral nerve, and that inhibition of PAD4 causes macrophages to accumulate early and in large numbers in the nerve parenchyma. Since the accumulation of macrophages in the nerve parenchyma can promote axonal regeneration, a substance that suppresses the expression or inhibits the function of PAD4 is useful for treating peripheral nerve injury.

The substance that suppresses the expression or inhibits the function of PAD4 used in the present disclosure is any substance having the ability to suppress the expression of PAD4 at the gene or protein level or having the ability to inhibit the function of PAD4, and is preferably at least one substance selected from the group consisting of a nucleic acid that suppresses the expression of PAD4, an antibody against PAD4 and antigen-binding fragment thereof, an aptamer targeting PAD4, and a PAD4 inhibitor.

One example of the substance that suppresses the expression of PAD4 is a nucleic acid that suppresses the expression of PAD4. The nucleic acid that suppresses the expression of PAD4 encompasses a nucleic acid having the ability to suppress transcription of mRNA from PAD4 gene (PAD4 mRNA), a nucleic acid having the ability to degrade PAD4 mRNA, and a nucleic acid having the ability to suppress protein translation from PAD4 mRNA. Examples include an antisense nucleic acid or a nucleic acid causing RNA interference, such as siRNA, shRNA, or miRNA, which can be designed and produced by those skilled in the art based on the nucleotide sequence of PAD4 gene or the nucleotide sequence of PAD4 mRNA.

One example of the substance that inhibits the function of PAD4 is an antibody against PAD4, preferably a specific antibody against PAD4, or an antigen-binding fragment of an antibody against PAD4. Another example of the substance that inhibits the function of PAD4 is an aptamer targeting PAD4. Definitions and other descriptions of antibodies, specific antibodies, antigen-binding fragments, and aptamers are as described above.

Another example of the substance that inhibits the function of PAD4 is a PAD4 inhibitor, which is any substance that has the ability to inhibit the function of PAD4. Examples of PAD4 inhibitor can include Cl-amidine (N²-Benzoyl-N⁵-(1-imino-2-chloroethyl)-L-ornithinamide trifluoroacetic acid), BMS-P5 (no generic name, Cas # 1550371-22-6), GSK121 trifluoroacetate (no generic name, Cas # 1652591-80-4).

In addition, DNA having the ability to induce transcription of the antisense nucleic acid or the nucleic acid causing RNA interference against any of the above-mentioned MIF, CXCR4, and PAD4 when positioned under the control of an appropriate expression promoter, and a nucleic acid in which a part of the nucleotide sequence of the antisense nucleic acid or the nucleic acid causing RNA interference is modified to enhance the stability against degradation by nucleases are also encompassed in the nucleic acids that suppress the expression in the present disclosure, as nucleic acids functionally equivalent to the antisense nucleic acid or the nucleic acid causing RNA interference. Examples of the modification include 2'O-methylation, 2'-fluorination, and 4'-thiolation.

Furthermore, a chimeric RNA in which a portion of ribonucleotides constituting RNA that suppresses the expression is substituted by corresponding deoxyribonucleotide(s) or nucleotide analog(s) is also encompassed in the nucleic acids that suppress the expression. Examples of the nucleotide analog include 5-position-modified uridine or cytidine, such as 5-(2-amino)propyluridine and 5-bromouridine; 8-position-modified adenosine or guanosine, such as 8-bromoguanosine; deaza-nucleotides, such as 7-deaza-adenosine; O- or N- alkylated nucleotides, such as N6-methyladenosine. The type or number of bases to be modified or substituted is not limited as long as the ability to suppress the expression of the target molecule is not lost.

The nucleic acids that suppress the expression can be artificially synthesized using genetic recombination or chemical synthesis techniques. Genetic recombination methods, chemical synthesis methods for nucleic acids, as well as techniques of synthesizing non-natural-type bases or techniques of synthesizing nucleic acids containing these are well known to those skilled in the art.

Further example of the substance that inhibits NETs formation by neutrophils can include lactoferrin as described in WO2014/168253; thrombin-like enzymes as described in WO2018/131724, such as batroxobin, ancrod, and crotalase; compounds with Btk inhibitory activity as described in WO2019/208805, such as tirabrutinib, ibrutinib, spebrutinib, acalablutinib, evobrutinib, poseltinib, fenebrutinib, vecablutinib, zanubrutinib, PRN-1008, and BMS-986142; immunoglobulins, such as sulfated human immunoglobulin G, at doses used in high dose immunoglobulin therapy (Uozumi et al., Modern Rheumatology, 2020, 30(3):544-550 . doi: 10.1080/14397595.2019.1602292.); PA-dPEG24 (PEG-modified peptide with a 24-mer PEG moiety added to the C-terminus of peptide: IALILEPICCQERAA) (Hair et al., PLoS One. 2019, 14(12):e 0226875. doi: 10.1371/journal.pone.0226875.); and Prostaglandin E2 (inhibition of NETosis through cAMP-PKA) (Shishikura et al., British Journal of Pharmacology, 2016, 173(2):319-31. doi: 10.1111/bph.13373).

### Substance that degrades NETs

The inventors have found that degradation of NETs causes macrophages to accumulate early and in large numbers in the parenchyma of an injured peripheral nerve, leading to the promotion of axonal regeneration. A substance that degrades NETs is useful in a pharmaceutical composition for treating peripheral nerve injury.

The substance that degrades NETs is any substance having the ability to degrade chromatin or antimicrobial proteins constituting NETs, particularly chromatin, examples of which include DNA degrading enzymes, such as DNase I, Staphylokinase (Thammavongsa et al., Science. 2013. 342(6160): 863-866. doi: 10.1126/science.1242255).

### Pharmaceutical Composition

In the present disclosure, the pharmaceutical composition contains the substance that suppresses the formation of NETs outside the parenchyma of an injured peripheral nerve in an amount effective for the treatment of peripheral nerve injury. The pharmaceutical composition contains, as an example, the substance that inhibits the accumulation of neutrophils in an injured peripheral nerve or the substance that suppresses the expression or inhibits the function of MIF, in an amount effective for the treatment of peripheral nerve injury. The amount effective for the treatment of peripheral nerve injury is determined as appropriate according to usage, subject age, gender, weight, site and degree of peripheral nerve injury, and other factors.

The pharmaceutical composition can contain at least one substance selected from the group consisting of the substance that inhibits the accumulation of neutrophils in an injured peripheral nerve, the substance that suppresses the expression of MIF, and the substance that inhibits the function of MIF. That is, the pharmaceutical composition may contain one or more substances that inhibit the accumulation of neutrophils in an injured peripheral nerve, may contain one or more substances that suppress the expression of MIF, and may contain one or more substances that inhibit the function of MIF.

Similarly, the pharmaceutical composition may contain one or more substances that suppress the expression of CXCR4, may contain one or more substances that inhibit the function of CXCR4, may contain one or more substances that suppress the expression of PAD4, may contain one or more substances that inhibit the function of PAD4, and may contain one or more substances that degrade NETs.

The pharmaceutical composition can contain other medical agents for the treatment of peripheral nerve injury, or pharmaceutically acceptable additives, such as a buffer, a stabilizer, a preservative, and an excipient. The pharmaceutically acceptable additives are well known to those skilled in the art and can be selected and used by those skilled in the art within the normal practice ability.

As used herein, the term "treatment" encompasses all types of medically acceptable therapeutic interventions aimed at cure or temporary remission of a disease or a condition. Thus, treatment of peripheral nerve injury encompasses medically acceptable interventions for a variety of purposes, including promotion of regeneration of an injured peripheral nerve, improvement of functional disorder caused by peripheral nerve injury, delay or stop of the progression of functional disorder, and prevention of the onset.

The pharmaceutical composition can be used for peripheral nerve injury where an axonal tear has occurred, and is particularly preferred for severe peripheral nerve injury where spontaneous recovery is not expected. Preferred examples of peripheral nerve injury that can be treated with the pharmaceutical composition are traumatic peripheral nerve injuries (wound, compression, traction, amputation, defect, medically-induced peripheral nerve injury, etc.).

The pharmaceutical composition is applied to subjects with peripheral nerve injury, for example, mammalian individuals such as rodents including mice, rats, hamsters, and guinea pigs, primates including humans, chimpanzees, and rhesus monkeys, domestic animals including pigs, cattle, goats, horses, and sheep, and pet animals including dogs and cats. Preferred subjects are humans.

The dosage form and administration route of the pharmaceutical composition are not limited as long as the pharmaceutical composition can suppress the formation of NETs outside the parenchyma of an injured peripheral nerve, for example, as long as it can inhibit the accumulation of neutrophils in an injured peripheral nerve, or it can suppress the expression or inhibit the function of MIF in an injured peripheral nerve.

For example, the pharmaceutical composition can be prepared in the form of a solution and administered intravenously or intraperitoneally. Alternatively, the pharmaceutical composition can be prepared in the form of a solution and placed in the distal vicinity of an injury site of a peripheral nerve, either as-is or impregnated in a biocompatible medical material. The placement may be made, for example, by injecting the solution agent into the distal vicinity of an injury site of a peripheral nerve, or by impregnating the solution agent into a biocompatible medical material and placing it in the distal vicinity of an injury site of a peripheral nerve.

The pharmaceutical composition can also be prepared in the form of a biocompatible medical material retaining the substance that suppresses the formation of NETs outside the parenchyma of an injured peripheral nerve, for example, the substance that inhibits the accumulation of neutrophils in an injured peripheral nerve or the substance that suppresses the expression or inhibits the function of MIF, and this biocompatible medical material can be used by placing in the distal vicinity of an injury site of a peripheral nerve. The method of retaining the substance in the biocompatible medical material is not limited, and for example, the substance may be fixed directly, fixed via an appropriate linker molecule or adapter molecule, or fixed using a binder or coating agent to the biocompatible medical material. The substance may be retained in the biocompatible medical material by nonspecific adsorption. When the biocompatible medical material is an absorbent material, the substance may be retained in the biocompatible medical material by impregnating the biocompatible medical material with a liquid containing the substance. Furthermore, when the biocompatible medical material is a gel-like material, the substance may be retained in the biocompatible medical material by allowing the substance to be contained in the raw material solution during the gel making process.

The pharmaceutical composition prepared in the form of a biocompatible medical material retaining the substance that suppresses the formation of NETs outside the parenchyma of an injured peripheral nerve, for example, the substance that inhibits the accumulation of neutrophils in an injured peripheral nerve or the substance that suppresses the expression or inhibits the function of MIF can also be represented as a medical material for the treatment of peripheral nerve injury retaining the substance that suppresses the formation of NETs outside the parenchyma of an injured peripheral nerve, for example, the substance that inhibits the accumulation of neutrophils in an injured peripheral nerve or the substance that suppresses the expression or inhibits the function of MIF.

Examples of the biocompatible medical material include polymer compounds such as polyfluoroethylene and polystyrene, inorganic compounds such as silica, and biodegradable polymers, and biodegradable polymers are preferred. Examples of the biodegradable polymers include synthetic polymer materials including polyglycolic acid, polylactic acid, polyethylene glycol, polycaprolactone, polydioxanone, and copolymers thereof, such as poly(lactic-co-glycolic acid); inorganic materials including β-tricalcium phosphate and calcium carbonate; and natural polymer materials including collagen, gelatin, alginate, hyaluronate, agarose, chitosan, fibrin, fibroin, chitin, cellulose, and silk.

The shape of the biocompatible medical material can be any shape suitable for placement in the distal vicinity of an injury site of a peripheral nerve, such as a sheet or gel. The medical material in sheet form can be used by covering the region in the distal vicinity of an injury site of a peripheral nerve. The medical material in gel form, such as fibrin glue, and alginate, can be used by spraying or applying it in the distal vicinity of an injury site of a peripheral nerve or by implanting it at the distal side of the injury site.

As shown in the Examples below, the pharmaceutical composition can promote axonal regeneration by being applied in the distal vicinity of an injury site of a peripheral nerve where Wallerian degeneration occurs, for a relatively short duration somewhere within the period beginning immediately after peripheral nerve injury and continuing through the accumulation and until the subsequent disappearance of neutrophils outside the nerve parenchyma distal to the injury site. Thus, the pharmaceutical composition is not required to exist over the entire injured peripheral nerve, nor is it required to exist over the entire period from immediately after peripheral nerve injury until the completion of axonal regeneration. In a preferred embodiment, the pharmaceutical composition can be used by being placed in the distal vicinity of, particularly in the distal close vicinity of an injury site of a peripheral nerve, for a relatively short duration, for example, for 30 minutes to 7 days, somewhere within the period of neutrophil accumulation outside the nerve parenchyma distal to the injury site after peripheral nerve injury, for example, somewhere within the period beginning immediately after peripheral nerve injury and continuing through the accumulation and until the subsequent disappearance of neutrophils outside the nerve parenchyma distal to the injury site.

For example, the pharmaceutical composition can be used by being placed in the distal vicinity of an injury site of a peripheral nerve, for a relatively short duration within the period beginning immediately, or 3, 6, or 9 hours after peripheral nerve injury and ending 12, 15, 18, 21, or 24 hours after peripheral nerve injury. The pharmaceutical composition can also be used by being placed in the distal vicinity of an injury site of a peripheral nerve, for a relatively short duration within the period from immediately to 24 hours, from 3 hours to 18 hours, from 6 hours to 15 hours, or from 9 hours to 12 hours after peripheral nerve injury.

When peripheral nerve reconstructions or sutures are performed some time after peripheral nerve injury, the surgical invasion by the reconstructions or sutures may cause neutrophil infiltration similar to that immediately after peripheral nerve injury. Thus, even if time has elapsed since the peripheral nerve injury, the treatment of peripheral nerve injury with the pharmaceutical composition is possible if the accumulation of neutrophils outside the nerve parenchyma is observed, similar to that immediately after peripheral nerve injury. In a preferred embodiment, the pharmaceutical composition can be used by being placed in the distal vicinity of, particularly in the distal close vicinity of an injury site of a peripheral nerve, for a relatively short duration, for example, for 30 minutes to 7 days, somewhere within the period of neutrophil accumulation outside the nerve parenchyma distal to the injury site after peripheral nerve injury, for example, somewhere within the period beginning immediately after surgical invasion and continuing through the accumulation and until the subsequent disappearance of neutrophils outside the nerve parenchyma distal to the injury site.

For example, the pharmaceutical composition can be used by being placed in the distal vicinity of an injury site of a peripheral nerve, for a relatively short duration within the period beginning immediately, or 3, 6, or 9 hours after surgical invasion and ending 12, 15, 18, 21, or 24 hours after surgical invasion. The pharmaceutical composition can also be used by being placed in the distal vicinity of an injury site of a peripheral nerve, for a relatively short duration within the period from immediately to 24 hours, from 3 hours to 18 hours, from 6 hours to 15 hours, or from 9 hours to 12 hours after surgical invasion.

The duration for placing the pharmaceutical composition in the distal vicinity of a peripheral nerve injury site can be, for example, 30 minutes or more, 60 minutes or more, or 90 minutes or more, and can be 7 days or less, 24 hours or less, 12 hours or less, 6 hours or less, 4 hours or less, or 3 hours or less.

In an illustrative embodiment, the pharmaceutical composition can be used by being placed in the distal vicinity of an injury site of a peripheral nerve for 30 minutes to 3 hours, within the period from 9 to 12 hours after peripheral nerve injury.

The pharmaceutical composition can be effective even with such temporally and spatially limited treatment. Therefore, for example, when performing nerve suture in cases of peripheral nerve injury, it is possible to place the pharmaceutical composition in the distal close vicinity of an injury site of a peripheral nerve and suture the nerve, and to remove the pharmaceutical composition after several tens of minutes to several hours, for example, 30 minutes to 3 hours, have elapsed since its placement.

In one aspect, the invention provides a method for treating peripheral nerve injury, including applying to a subject with peripheral nerve injury the pharmaceutical composition containing an effective amount of the substance that suppresses the formation of NETs outside the parenchyma of an injured peripheral nerve, for example, the substance that inhibits the accumulation of neutrophils in an injured peripheral nerve or the substance that suppresses the expression or inhibits the function of MIF. In one embodiment, the application is performed by placement of the pharmaceutical composition preferably in the distal vicinity of, particularly in the distal close vicinity of an injury site of a peripheral nerve. In one aspect, the invention also provides a substance that suppresses the formation of NETs outside the parenchyma of an injured peripheral nerve for use in the treatment of peripheral nerve injury. In one aspect, the invention further provides use of the substance that suppresses the formation of NETs outside the parenchyma of an injured peripheral nerve in the manufacture of the pharmaceutical composition. Definitions and other descriptions are as described above.

### Evaluation method

In one aspect, the present invention provides a method for evaluating the peripheral nerve regeneration-promoting activity of a test substance by using its activity of suppressing NETs formation outside the parenchyma of an injured peripheral nerve as an indicator. The activity of suppressing NETs formation outside the parenchyma of an injured peripheral nerve includes the activity of inhibiting the accumulation of neutrophils in an injured peripheral nerve; the activity of inhibiting NETs formation by neutrophils, for example, the activity of suppressing the expression or inhibiting the function of MIF, the activity of suppressing the expression or inhibiting the function of CXCR4, and the activity of suppressing the expression or inhibiting the function of PAD4; and the activity of degrading NETs.

In one embodiment, the method for evaluation by using the activity of inhibiting the accumulation of neutrophils in an injured peripheral nerve as an indicator includes determining the peripheral nerve regeneration-promoting activity of a test substance by comparing the viability or migration ability of neutrophils in vitro, both in the presence and absence of the test substance. In this embodiment, the test substance that reduces the viability or migration ability of neutrophils can be determined to have peripheral nerve regeneration promoting activity.

In another embodiment, the method for evaluation by using the activity of inhibiting the accumulation of neutrophils in an injured peripheral nerve as an indicator includes determining the peripheral nerve regeneration-promoting activity of a test substance by comparing the number of neutrophils in the blood or the number of neutrophils accumulated in an injury site of peripheral nerve in vivo, both in the presence and absence of the test substance. In this embodiment, the test substance that reduces the number of neutrophils in the blood or the number of neutrophils accumulated in an injury site of peripheral nerve can be determined to have peripheral nerve regeneration-promoting activity.

In one embodiment, the method for evaluation by using the activity of inhibiting NETs formation by neutrophils as an indicator includes determining the peripheral nerve regeneration-promoting activity of a test substance in vitro by comparing the promoter activity of either MIF, CXCR4 or PAD4 gene (hereinafter referred to as the indicator gene) in vitro, both in the presence and absence of the test substance. In this embodiment, the evaluation method can include, for example, the step of incubating the test substance together with cells containing a nucleic acid having a marker gene under the control of a promoter region that controls expression of the indicator gene, under a condition where expression of the marker gene is induced by said promoter; the step of measuring the intensity of the marker gene-derived signal in said cells; the step of comparing the above measured intensity of the marker gene-derived signal with the intensity of the marker gene-derived signal in the absence of the test substance; and the step of determining that the test substance that reduces the intensity of the marker gene-derived signal has peripheral nerve regeneration-promoting activity.

The marker gene can be any gene whose expression induction can be detected, such as a gene encoding a fluorescent protein such as GFP or a chemiluminescent protein such as luciferase, or a gene that confers a phenotypic change on cells, such as a drug resistance gene or a gene that can complement the nutritional requirements of cells.

The cells containing a nucleic acid having a marker gene under the control of a promoter region that controls expression of the indicator gene can be produced, for example, by recombining the nucleotide sequence of the open reading frame region of the indicator gene in a cell that originally has the indicator gene with the nucleotide sequence that encodes the marker gene. The cells can also be produced by constructing an expression vector containing the promoter region of the human indicator gene and the recombined marker gene under its control based on the human genome sequence information, and introducing the expression vector into appropriate cells.

Incubation can be performed by allowing said cells and the test substance to coexist in a medium or buffer solution in an appropriate container. The temperature, time, and other conditions of incubation can be any conditions under which the cells can express the marker gene. The marker gene-derived signal can be measured by a method suitable for the marker gene used.

In a further embodiment, the evaluation method includes determining the peripheral nerve regeneration-promoting activity of the test substance by comparing the expression of the indicator gene or either MIF, CXCR4 or PAD4 protein (hereinafter referred to as the indicator protein) both in the presence and absence of the test substance. In this embodiment, the evaluation method can include, for example, the step of incubating the test substance together with cells having the ability to express the indicator gene or indicator protein; the step of measuring the intensity of the indicator gene- or indicator protein-derived signal in said cells after incubation; the step of comparing the above measured intensity of the indicator gene- or indicator protein-derived signal with the intensity of the indicator gene- or indicator protein-derived signal in the absence of the test substance; and the step of determining that the test substance that reduces the intensity of the indicator gene or indicator protein-derived signal has peripheral nerve regeneration-promoting activity.

The cells having the ability to express the indicator gene or indicator protein are preferably cells expressing a human indicator gene or indicator protein, for example, a cultured cell line incorporating a human indicator gene, for example, a human MIF gene, can be used.

Incubation can be performed by coexistence of said cells and the test substance in a medium or buffer solution in an appropriate container. The temperature, time, and other conditions of incubation can be any conditions under which the cells can express the indicator gene or indicator protein.

The indicator gene-derived signal can be measured by general methods that can detect and quantify specific gene expression, such as hybridization using the nucleotide sequence information of the indicator gene, quantitative PCR, and RNA-Seq. The indicator protein-derived signal can be measured by general methods that can detect and quantify specific protein expression, for example, immunological detection methods such as ELISA and Western blotting using a specific antibody against the indicator protein.

The test substance that reduces the intensity of the indicator gene or indicator protein-derived signal in cells incubated with the test substance compared to cells not incubated with the test substance under conditions that the expression of the indicator gene or indicator protein is induced, can be determined to have peripheral nerve regeneration-promoting activity.

In another embodiment, the method for evaluation by using the activity of inhibiting NETs formation by neutrophils as an indicator includes determining the peripheral nerve regeneration-promoting activity of the test substance by comparing the function of MIF, CXCR4, or PAD4 both in the presence and absence of the test substance. For example, the method for evaluation by using the activity of inhibiting NETs formation by neutrophils as an indicator includes determining the peripheral nerve regeneration-promoting activity of the test substance by comparing the inhibitory activity of MIF on macrophage migration both in the presence and absence of the test substance. In this embodiment, the test substance that is determined to have activity to inhibit the function of MIF can be evaluated as having peripheral nerve regeneration-promoting activity. Comparison of the activity of MIF both in the presence and absence of the test substance can be performed using methods employed in the evaluation or screening of the inhibitory activity of known MIF inhibitors.

In yet another embodiment, the evaluation method by using the activity of degrading NETs as an indicator includes determining the peripheral nerve regeneration-promoting activity of the test substance by comparing the degradation of NETs both in the presence and absence of the test substance. In this embodiment, the evaluation method can include, for example, the step of incubating the test substance together with NETs formed by neutrophils; the step of measuring the intensity of the NETs-derived signal after incubation; the step of comparing the above measured intensity of the NETs-derived signal with the intensity of NETs-derived signal in the absence of the test substance; and the step of determining that the test substance that reduces the intensity of the NETs-derived signal has peripheral nerve regeneration-promoting activity. The NETs-derived signal is, for example, a signal derived from a known NETs marker molecule such as citrullinated histone H3, and can be measured by methods suitable for the marker molecule, such as immunological detection methods. In this embodiment, the test substance that is determined to have activity to degrade NETs can be evaluated as having peripheral nerve regeneration-promoting activity.

While the invention will be described in more detail by the following examples, it should not be considered as being limited to these examples.

### [Examples]

### Materials and Methods

### (1) Animal experiments

Anesthetized Lewis rats (males, 12-16 weeks old) or C57BL/6 mice (males, 8-10 weeks old) were placed in the prone position and a longitudinal skin incision was made from the left buttock to the distal left thigh to expose the entire sciatic nerve. Crush injuries were created with micro mosquito forceps (Fine Science Tools, No. 13010-12). An 8-0 Nylon thread was applied to the epineurium at the crush injury for marking, and the incision was closed. In Test Example 2 and Examples 1-4, the animals were treated as described after the creation of the crush injury.

After a predetermined time had elapsed from the creation of the crush injury, the right atrial appendage of the animals was incised under anesthesia to release the blood, and a PBS solution was poured into the left heart to remove the blood. After transcardial perfusion and fixation with 4% paraformaldehyde-added PBS, the sciatic nerve was collected.

### (2) Preparation of GFP-labeled neutrophils

GFP-labeled neutrophils used in Test Example 2 were prepared as follows: Bone marrow cells from the femur and lower leg bones of C57BL/6 GFP mice (8-10 weeks old) were collected by draining them with PBS. Erythrocytes contained in bone marrow cells were lysed with RBC lysis buffer (Funakoshi) to purify bone marrow cells. Neutrophils were isolated from bone marrow cells using Neutrophil isolation kit, mouse (Miltenyi Biotec).

### (3) Immunohistochemical staining

Sciatic nerves collected from animals were fixed in 4% PFA at 4°C overnight and then transferred to 30% Sucrose/PB solution and allowed to stand for 24 hours or longer. Longitudinal sciatic nerve sections of 10 µm thickness, or in the case of mice, transverse sections at predetermined locations, were prepared using a cryostat. The sections were blocked in TBS (pH 8.4) containing 5% Normal horse serum (Thermo Fisher Scientific, Waltham, MA) and 0.25% Triton X-100 (Sigma-Aldrich, St. Louis, MO) for 1 hour, and then immersed in TBS containing primary antibody at 4°C overnight and in TBS containing secondary antibody at room temperature for 1 hour for staining. After washing with TBS, counterstaining was performed with DAPI. After staining, tissues were observed using a fluorescence microscope BZ-X710 (KEYENCE). Image analysis was performed using Image J (Schneider et al., 2012).

### Primary antibodies used

Neutrophil markers: Ly-6G (rat anti-Ly6G antibody, Gentex Cat# GTX40913),
Neutrophil elastase (NE) (rabbit, anti-neutrophil elastase antibody, Abcam, Cat # ab68672)
Macrophage marker: CD68 (mouse, anti-CD68 antibody, Bio-rad, Cat # MCA341R)
Myelin sheath marker: MBP (chicken Anti-MBP antibody, Aves lab, Cat # AB_2313550)
Axon marker: β3 tubulin (rabbit, anti-β3 tubulin antibody, Covance, Cat # PRB-435P)
NETs marker: citrullinated histone H3 (CitH3) (rabbit, anti-CitH3 antibody, Abcam # ab5103)
Type I collagen (rabbit, anti-type1 collagen antibody, Southernbiotech, Cat # 1310-01)
MIF (rabbit, anti-MIF antibody, Thermo Scientific, Cat # PA527343) CXCR4 (rabbit, anti-CXCR4 antibody, Novus # NB100-56437)
Secondary antibodies were selected according to the host animal species and molecular species of the primary antibody, taking multiplex staining into consideration.

### (4) Axonal regeneration

In β3 tubulin-immunostained images of longitudinal sciatic nerve sections, vertical lines were set at 5 mm intervals in the direction distal to the injury site, and the number of axons crossing each line was divided by the length of the line to calculate axon density. The axonal regeneration percentage was calculated by dividing the axon density at each location by the axon density at uninjured site 1.5 mm proximal to the injury site. In Example 2, axon density was calculated by dividing the number of axons in the β3 tubulin-immunostained image of a transverse sciatic nerve section by the area of the section.

### (5) Statistics

Experimental data were expressed as mean ± standard error. Statistical analysis was performed using the statistical analysis software JMP Pro 11.0 (SAS Institute). Student's t-test was used for comparisons between two groups. Tukey test was used for comparisons among multiple groups. p-value of less than 0.05 was considered significant.

### Test Example 1: Spatiotemporal distribution analysis of neutrophils and macrophages in peripheral nerve injury area

### (1) Neutrophils

Crush injuries were created in the sciatic nerve of rats, the sciatic nerves were collected 12 hours later, and their longitudinal sections were subjected to HE staining and immunohistochemical staining. Representative HE-stained images from 5 mm proximal (-5 mm) to 25 mm distal to the injury site are shown in Figure 1 and immunostained images in Figure 2. Neutrophils were observed both in the parenchyma and outside the parenchyma ("outside the parenchyma" is used hereafter synonymously with "epineurium") at the injury site and 5 mm distal to the injury site, whereas in the Wallerian degeneration region at 15 mm and 25 mm distal to the injury site, neutrophils were absent in the parenchyma and present only outside the parenchyma.

The number of neutrophils in the nerve (including both parenchymal and outside the parenchyma) was counted from HE-stained images, and the spatial distribution of neutrophils was examined (n=3). Neutrophils were found to be distributed in large numbers in the region of Wallerian degeneration distal to the injury site (Fig. 3).

To further examine the temporal distribution of neutrophils, sciatic nerves were collected from rats with crush injuries at 1 hour, 6 hours, 12 hours, 1 day, 3 days, or 7 days after injury creation, and immunohistochemical staining was performed as described above to count the number of neutrophils in the nerve (n=3). The increase in the number of neutrophils in the distal regions to the injury site peaked at 12 hours after injury and had almost disappeared by 3 days (Fig. 4).

### (2) Macrophages

Immunohistochemical staining for CD68, a macrophage marker, was performed on longitudinal sections of sciatic nerves collected from rats 7 days after crush injury creation. Representative immunostained images from 5 mm to 30 mm distal to the injury site are shown in Figure 5. CD68-positive cells were uniformly distributed in the nerve parenchyma where Wallerian degeneration occurred.

To examine the temporal distribution of macrophages, sciatic nerves were collected from rats with crush injuries at 1 hour, 6 hours, 12 hours, 1 day, 3 days, or 7 days after injury creation, and immunohistochemical staining of the sciatic nerves was performed in the same way as above to count CD68-positive cells present in the nerve parenchyma as macrophages. The number of macrophages in the distal regions to the injury site increased from 3 days after injury and peaked at 7 days (Figure 6). Macrophages accumulated in the epineurium as well as neutrophils 12 hours after injury (Fig. 7).

### Example 1: Evaluation of effect of anti-neutrophil antibodies on nerve regeneration process

Anti-polymorphonuclear (PMN) antibody (rabbit anti-rat PMN antibody , CEDARLANE, Cat AIAD51140) at 1 ml/kg or control antibody at 0.1 ml/100 g body weight was administered intraperitoneally to rats (n=3), and 3 hours later, crush injuries were created in their sciatic nerves. A control group that underwent only the creation of crush injury was also prepared (n=3). After the intraperitoneal administration, blood was collected over time and Giemsa stained, and the percentage of polymorphonuclear cells to the total number of stained nucleated cells was calculated as the number of neutrophils in the blood (%). The sciatic nerves were collected 12 hours or 7 days after injury creation, and their longitudinal sections were subjected to HE staining and immunohistochemical staining. The number of neutrophils in the nerve and macrophages in the nerve parenchyma were counted from the stained images, and the axonal regeneration percentage was calculated. The MBP positive ratio, an index of the amount of axonal debris, was calculated by dividing the area of MBP positive regions in the MBP-immunostained images by the total area.

Figure 8 shows the change over time in the number of neutrophils in the blood. The number of neutrophils in the blood increased and peaked at 12 hours after injury in the injury alone group and the control antibody group, indicating the mobilization of neutrophils into the circulating pool. On the other hand, in the anti-neutrophil antibody group, the number of neutrophils in the blood did not change greatly after injury.

Figure 9 shows representative HE-stained images at 5 mm distal to the injury site 12 hours after injury, and Figure 10 shows the distribution of neutrophils in the distal regions to the injury site. The number of neutrophils observed in the distal regions to the injury site in the anti-neutrophil antibody group was lower than in the injury alone group and the control antibody group. These results demonstrated that anti-neutrophil antibodies reduced the number of neutrophils both in the blood and in the Wallerian degeneration region.

Figure 11 shows the distribution of macrophages in the distal regions to the injury site of sciatic nerve 12 hours and 7 days after injury. The anti-neutrophil antibody group showed a greater accumulation of macrophages at an earlier time point than the injury alone group and the control antibody group.

Figure 12 shows representative β3 tubulin-immunostained images from 5 mm to 30 mm distal to the injury site 7 days after injury, and Figure 13 shows the axonal regeneration percentage. The anti-neutrophil antibody group showed a higher axonal regeneration percentage than the injury alone group and the control antibody group.

Figure 14 shows representative MBP-immunostained images and MBP positive ratio 7 days after injury. The anti-neutrophil antibody group showed a lower MBP positive ratio and a reduced amount of myelin debris compared to the injury alone group and the control antibody-treated group.

This example demonstrated that the decrease in the number of neutrophils accumulated in the Wallerian degeneration region by anti-neutrophil antibodies increased the accumulation of macrophages, promoted axonal regeneration, and enhanced clearance of myelin.

### Test Example 2: Evaluation of effect of increased number of neutrophils on nerve regeneration process

Crush injuries were created in the sciatic nerve of mice, and 11 hours later, 2 × 10⁶ GFP-labeled neutrophils suspended in PBS per animal or the same volume of PBS alone was administered intravenously (n = 3). A control group that underwent only the creation of crush injury was also prepared (n=3). Blood was collected 12 hours after injury creation and Giemsa stained to calculate the number of neutrophils in the blood. The sciatic nerves were collected 12 hours or 7 days after injury creation, and their transverse sections at 4 mm, 6 mm, 8 mm, and 10 mm distal to the injury site were subjected to HE staining and immunohistochemical staining. The number of neutrophils in the nerve and the number of macrophages in the nerve parenchyma were counted, and the axon density and MBP positive ratio were calculated.

Figure 15 shows the number of neutrophils in the blood at 12 hours after injury, Figure 16 shows representative HE-stained and immunostained images at 4 mm distal to the injury site, and Figure 17 shows the spatial distribution of neutrophils. The number of neutrophils in the blood was increased in the neutrophil group compared to the injury alone group and the PBS group, and the number of neutrophils in the distal regions to the injury site was also increased. Neutrophils, including intravenously administered GFP-positive neutrophils, accumulated outside the nerve parenchyma in the distal regions to the injury site.

Figure 18 shows the distribution of macrophages in the distal regions to the injury site of sciatic nerve 7 days after injury. The number of macrophages in the distal regions to the injury site was lower in the neutrophil group than in the injury alone group and the PBS group.

Figure 19 shows representative β3 tubulin-immunostained images, and Figure 20 shows axon density 7 days after injury. In the injury alone group and the PBS group, axon density increased closer to the injury site, while axon density was generally lower in the neutrophil group.

Figure 21 shows representative MBP-immunostained images 7 days after injury, and Figure 22 shows the MBP positivity ratio. The neutrophil group showed a higher MBP positive ratio and an increased amount of myelin debris compared to the injury alone group and the PBS group.

This test example demonstrated that neutrophil administration increased the number of neutrophils accumulating in the Wallerian degeneration region, which was accompanied by suppression of macrophage accumulation, axonal regeneration, and myelin clearance.

A conceptual diagram of axonal regeneration process controlled by neutrophils, as inferred from Test Examples 1 and 2 and Example 1, is shown in Figure 23.

Example 2: Evaluation of NETs formation in epineurium of Wallerian degeneration region and effect of suppression of NETs formation on nerve regeneration process

### (1) Distribution of NETs

Immunohistochemical staining for CitH3, Ly-6G, and CD68 was performed on longitudinal sections of the sciatic nerve collected from untreated rats (intact sciatic nerve) and the sciatic nerve collected from rats 12 hours after creation of crush injury (injured nerve) at 20 mm distal to the injury site. No NETs were detected in the intact sciatic nerve (Fig. 24 left), whereas large amounts of NETs were detected in the epineurium of the Wallerian degeneration region of the injured nerve (Fig. 24 center and right). NETs in the epineurium co-localized with neutrophils. This suggests that NETs were formed by neutrophils accumulated in the epineurium of the Wallerian degeneration region.

(2) Study on coverage of distal region to nerve injury site (from 15 mm to 25 mm distal to injury site) using sheet containing NETs formation inhibitor or NETs-degrading enzyme

Crush injuries were created in the sciatic nerve of rats. Ten hours after injury creation, the sciatic nerves from 15 mm to 25 mm distal to the injury site were wrapped with the collagen-based artificial skin PELNAC (registered trademark) (Smith & Nephew; hereafter also referred to as sheets or collagen sheets) impregnated with either 50 µl of PBS containing 0.3 mg of the PAD4 inhibitor Cl-amidine (Cayman CHEMICAL #10599), 50 µl of PBS containing 200 U of DNase I (Nippon Gene #314-08071), or 50 µl of PBS (control). The sheets were suture-fixed with 8-0 nylon and placed for 2 hours (n = 3 in each group). Twelve hours after injury creation, the sciatic nerves were collected, their longitudinal sections at 20 mm distal to the injury site were prepared and immunohistochemical staining for CitH3, Ly-6G, and CD68 was performed. The epineural area was observed and the percentage of area stained for CitH3 in the total area of the field of view was calculated. The number of macrophages per unit area of the nerve parenchyma was quantified.

Figure 25 shows representative CitH3 and Ly-6G-immunostained images and the CitH3 positive area, and Figure 26 shows representative CD68-immunostained images and the number of macrophages. A large amount of NETs were observed in the epineurium in the control sheet group, while only a small amount of NETs were detected in the Cl-amidine-containing sheet group and the DNase I-containing sheet group. In addition, more macrophages accumulated in the nerve parenchyma in the Cl-amidine-containing sheet group and the DNase I-containing sheet group than in the control sheet group.

(3) Study on coverage of distal region to nerve injury site (from 5 mm to 25 mm distal to injury site) using sheet containing NETs-degrading enzyme

Crush injuries were created in the sciatic nerve of rats. Ten hours after injury creation, the sciatic nerves from 5 mm to 25 mm distal to the injury site were wrapped with collagen sheets impregnated with either 100 µl of PBS containing 400 U DNase I (Nippon Gene #314-08071), or 100 µl of PBS (control). The sheets were suture-fixed with 8-0 nylon and placed for 2 hours (n = 1 in each group). Seven days after injury creation, the sciatic nerves were collected, and immunohistochemical staining of their longitudinal sections was performed to count the number of macrophages in the nerve parenchyma, and the axonal regeneration percentage was calculated.

Representative β3 tubulin-immunostained images are shown in the upper panels of Figure 27, the axonal regeneration percentage in the lower panel of Figure 27, and the number of macrophages in Figure 28. The DNase I-containing sheet group showed higher axonal regeneration percentage and a greater accumulation of macrophages in the nerve parenchyma compared to the control sheet group over almost the entire evaluated regions distal to the injury site.

These results demonstrated that axonal regeneration is promoted by inhibiting the formation of NETs in the epineurium of the Wallerian degeneration region of the injured nerve.

Example 3: Evaluation of effect of MIF inhibition on nerve regeneration process

### (1) Distribution of MIF

Immunohistochemical staining for MIF and Ly-6G was performed on longitudinal sections of the sciatic nerve collected from untreated rats and the sciatic nerve collected from rats 12 hours after creation of crush injury. No MIF expression was observed in the intact sciatic nerve (Figure 29, upper panels), whereas MIF expression was observed outside the parenchyma of the sciatic nerve 12 hours after injury and MIF was double-stained with neutrophils (Figure 29, lower panels). This suggests that MIF is produced by accumulated neutrophils outside the parenchyma in the Wallerian degeneration region 12 hours after injury.

### (2) Study on coverage of distal region to nerve injury site (from 15 mm to 25 mm distal to injury site) using sheet containing MIF inhibitor

Crush injuries were created in the sciatic nerve of rats. Ten hours after injury creation, the sciatic nerves from 15 mm to 25 mm distal to the injury site were wrapped with collagen sheets impregnated with either 50 µl of 1% DMSO containing 500 µg of the MIF inhibitor iso-1 (Abcam, Cat ab142140), or 50 µl of 1% DMSO. The sheets were suture-fixed with 8-0 nylon and placed for 2 hours (n = 3 in each group). Twelve hours after injury creation, the sciatic nerves were collected, their longitudinal sections were prepared, and immunohistochemical staining for CitH3, Ly-6G, and CD68 was performed. The epineural area was observed and the percentage of area stained for CitH3 in the total area of the field of view was calculated. The number of macrophages per unit area of the nerve parenchyma was quantified.

Figure 30 shows CD68-immunostained images and the number of macrophages at 5 mm distal to the injury site of the sciatic nerve 12 hours after injury, Figure 31 shows CD68-immunostained images and the number of macrophages at 20 mm distal to the injury site of the sciatic nerve, and Figure 32 shows CitH3 and Ly-6G-immunostained images and CitH3-positive area at 20 mm distal to the injury site of the sciatic nerve. There was no difference in macrophage accumulation between the iso-1-containing sheet group and the control sheet group at 5 mm distal to the injury site, which did not receive the sheet treatment. On the other hand, at the 20 mm distal to the injury site, which received the sheet treatment, more macrophages accumulated in the nerve parenchyma in the iso-1-containing sheet group than in the control sheet group. In the iso-1-containing sheet group, only a small amount of NETs were detected in the epineurium at 20 mm distal to the injury site.

### (3) Study on coverage of distal region to nerve injury site (from 5 mm to 25 mm distal to injury site) using sheet containing MIF inhibitor

Crush injuries were created in the sciatic nerve of rats. Ten hours after injury creation, the sciatic nerves from 5 mm to 25 mm distal to the injury site were wrapped with PELNAC (registered trademark) impregnated with either 100 µl of 1% DMSO containing 1000 µg iso-1, or 100 µl of 1% DMSO, and the sheets were suture-fixed and placed for 2 hours (n = 3 in each group). The sheets were removed 12 hours after injury creation and the incisions were closed after washing. Seven days after injury creation, the sciatic nerves were collected, and immunohistochemical staining of their longitudinal sections was performed to count the number of macrophages in the nerve parenchyma, and the axonal regeneration percentage was calculated.

Figure 33 shows representative CD68-immunostained images and the number of macrophages, and Figure 34 shows CD163-immunostained images and the number of M2 macrophages. Over the entire evaluated regions distal to the injury site, there was a greater accumulation of macrophages in the nerve parenchyma in the iso-1-containing sheet group compared to the control sheet group.

Figure 35 shows representative β3 tubulin-immunostained images and axonal regeneration percentage. The iso-1-containing sheet group showed a higher axonal regeneration percentage than the control sheet group over almost the entire evaluated regions distal to the injury site.

### (4) Study on coverage of distal region to nerve injury site (from 5 mm to 15 mm distal to injury site) using sheet containing MIF inhibitor

Crush injuries were created in the sciatic nerve of rats. Ten hours after injury creation, the sciatic nerves from 5 mm to 15 mm distal to the injury site were wrapped with PELNAC (registered trademark) impregnated with either 50 µl of 1% DMSO containing 500 µg iso-1, 50 µl of 1% DMSO containing 500 µg of the MIF inhibitor CPSI-1306 (MCH, Cat# HY110095), or 50 µl of 1% DMSO, and the sheets were suture-fixed and placed for 2 hours (n = 1 in each group). The sheets were removed 12 hours after injury creation and the incisions were closed after washing. Seven days after injury creation, the sciatic nerves were collected, and immunohistochemical staining of their longitudinal sections was performed to calculate the axonal regeneration percentage.

Figure 36 shows representative β3 tubulin-immunostained images, and Figure 37 shows the axonal regeneration percentage and longest axon length. The iso-1 containing sheet group and CPSI-1306 containing sheet group showed higher axonal regeneration percentage and greater longest axon length than the control sheet group over almost the entire evaluated regions distal to the injury site.

These results demonstrated that axonal regeneration was promoted by inhibiting MIF outside the parenchyma in the Wallerian degeneration region.

### Example 4: Evaluation of effect of CXCR4 inhibition on nerve regeneration process

### (1) Distribution of CXCR4

Immunohistochemical staining for CXCR4 and Ly-6G was performed on longitudinal sections of the sciatic nerve collected from untreated rats and the sciatic nerve collected from rats 12 hours after creation of crush injury. No CXCR4 expression was observed in the intact sciatic nerve, whereas CXCR4 expression was observed outside the parenchyma of the sciatic nerve 12 hours after injury and CXCR4 was double-stained with neutrophils (Figure 38). This suggests that CXCR4 is produced by accumulated neutrophils outside the parenchyma in the Wallerian degeneration region 12 hours after injury.

### (2) Study on coverage of distal region to nerve injury site (from 15 mm to 25 mm distal to injury site) using sheet containing CXCR4 inhibitor

Crush injuries were created in the sciatic nerve of rats. Ten hours after injury creation, the sciatic nerves from 15 mm to 25 mm distal to the injury site were wrapped with collagen sheets impregnated with either 50 µl of PBS containing 500 µg of the CXCR4 inhibitor AMD3100 (Abcam #10599), or 50 µl of PBS. The sheets were suture-fixed with 8-0 nylon and placed for 2 hours (n = 3 in each group). Twelve hours after injury creation, the sciatic nerves were collected, their longitudinal sections at 20 mm distal to the injury site were prepared, and immunohistochemical staining for CitH3, Ly-6G, and CD68 was performed. The epineural area was observed and the percentage of area stained for CitH3 in the total area of the field of view was calculated. The number of macrophages per unit area of the nerve parenchyma was quantified.

Figure 39 shows representative CitH3 and Ly-6G-immunostained images and CitH3-positive area, and Figure 40 shows representative CD68-immunostained images and the number of macrophages. In the AMD3100-containing sheet group, only a small amount of NETs were detected in the epineurium, and more macrophages accumulated in the nerve parenchyma than in the control sheet group.

### (3) Study on coverage of distal region to nerve injury site (from 5 mm to 25 mm distal to injury site) using sheet containing CXCR4 inhibitor

Crush injuries were created in the sciatic nerve of rats. Ten hours after injury creation, the sciatic nerves from 5 mm to 25 mm distal to the injury site were wrapped with collagen sheets impregnated with either 100 µl of PBS containing 1 mg of the CXCR4 inhibitor AMD3100 (Abcam #10599), or 100 µl of PBS. The sheets were suture-fixed with 8-0 nylon and placed for 2 hours (n = 1 in each group). Seven days after injury creation, the sciatic nerves were collected, and immunohistochemical staining of their longitudinal sections was performed to count the number of macrophages in the nerve parenchyma, and the axonal regeneration percentage was calculated.

Representative β3 tubulin-immunostained images are shown in the upper panels of Figure 41, the axonal regeneration percentage is shown in the lower panel of Figure 41, and the number of macrophages is shown in Figure 42. In the CXCR4-containing sheet group, a higher axonal regeneration percentage was observed, and more macrophages accumulated in the nerve parenchyma than in the control sheet group over almost the entire evaluated regions distal to the injury site.

A conceptual diagram of axonal regeneration process controlled by neutrophils, as inferred from Test Examples 1 and 2 and Examples 1 to 4, is shown in Figure 43.

### Sequence Listing Free Text

SEQ ID NO: 1 Amino acid sequence of the peptide portion in PA-dPEG24

## Claims

1. A pharmaceutical composition for treating peripheral nerve injury, comprising a substance that suppresses the formation of neutrophil extracellular traps (NETs) outside the parenchyma of an injured peripheral nerve.

2. The pharmaceutical composition according to claim 1, wherein the substance that suppresses the formation of NETs outside the parenchyma of an injured peripheral nerve is a substance that inhibits the accumulation of neutrophils in an injured peripheral nerve, a substance that inhibits NETs formation by neutrophils, or a substance that degrades NETs.

3. The pharmaceutical composition according to claim 2, wherein the substance that inhibits the accumulation of neutrophils in an injured peripheral nerve is selected from the group consisting of an antibody against neutrophils and antigen-binding fragment thereof, an aptamer targeting neutrophils, an inhibitor of neutrophil migration stimulating factor, and a neutrophil migration inhibitory factor.

4. The pharmaceutical composition according to claim 2, wherein the substance that inhibits NETs formation by neutrophils is selected from the group consisting of a substance that suppresses the expression or inhibits the function of macrophage migration inhibitory factor (MIF), a substance that suppresses the expression or inhibits the function of CXC motif-type chemokine receptor 4 (CXCR4), and a substance that suppresses the expression or inhibits the function of peptidylarginine deiminase (PAD4).

5. The pharmaceutical composition according to claim 4, wherein the substance that suppresses the expression or inhibits the function of MIF is selected from the group consisting of a nucleic acid that suppresses the expression of MIF, an antibody against MIF and antigen-binding fragment thereof, an aptamer targeting MIF, and a MIF inhibitor.

6. The pharmaceutical composition according to claim 5, wherein the MIF inhibitor is ISO-1((S,R)-3-(4-Hydroxyphenyl)-4,5-dihydro-5-isoxazole acetic acid) or (±)-CPSI 1306(2-[3-(3-(2,4-Difluorophenyl)-4,5-dihydro-5-isoxazolyl]-1-(4-morpholinyl)ethanone).

7. The pharmaceutical composition according to claim 4, wherein the substance that suppresses the expression or inhibits the function of CXCR4 is selected from the group consisting of a nucleic acid that suppresses the expression of CXCR4, an antibody against CXCR4 and antigen-binding fragment thereof, an aptamer targeting CXCR4, and a CXCR4 inhibitor.

8. The pharmaceutical composition according to claim 7, wherein the CXCR4 inhibitor is plerixafol.

9. The pharmaceutical composition according to claim 4, wherein the substance that suppresses the expression or inhibits the function of PAD4 is selected from the group consisting of a nucleic acid that suppresses the expression of PAD4, an antibody against PAD4 and antigen-binding fragment thereof, an aptamer targeting PAD4, and a PAD4 inhibitor.

10. The pharmaceutical composition according to claim 9, wherein the PAD4 inhibitor is Cl-amidine.

11. The pharmaceutical composition according to claim 2, wherein the substance that degrades NETs is DNase I.

12. The pharmaceutical composition according to any one of claims 1-11, for use by placement in the distal vicinity of an injury site of a peripheral nerve.

13. A method for evaluating the peripheral nerve regeneration-promoting activity of a test substance by using the activity of the substance of suppressing NETs formation outside the parenchyma of an injured peripheral nerve as an indicator.

14. The method according to claim 13, wherein the activity of the substance of suppressing NETs formation outside the parenchyma of an injured peripheral nerve is the activity of inhibiting the accumulation of neutrophils in an injured peripheral nerve, the activity of inhibiting NETs formation by neutrophils, or the activity of degrading NETs.

15. The method according to claim 14, wherein the activity of inhibiting NETs formation by neutrophils is selected from the group consisting of the activity of suppressing the expression or inhibiting the function of MIF, the activity of suppressing the expression or inhibiting the function of CXCR4, and the activity of suppressing the expression or inhibiting the function of PAD4.
